# EUROPEAN PATENT APPLICATION

(11) **EP 4 785 852 A1**
(43) Date of publication of application: **05.08.2026**
(21) Application number: 24871827.2
(22) Date of filing: 10.09.2024
(51) Int. Cl.: A61B 3/10

(54) **OPTICAL COHERENCE TOMOGRAPHY DEVICE, CONTROL METHOD THEREFOR, AND PROGRAM**

(30) Priority: 27.09.2023 JP 2023165916
(71) Applicant: Topcon Corporation, Tokyo 174-8580 (JP)
(72) Inventor: MORIGUCHI Yoshikiyo, Tokyo 174-8580 (JP); ISHIKAWA Akiko, Tokyo 174-8580 (JP); MINO Toshihiro, Tokyo 174-8580 (JP); TAMURA Masato, Tokyo 174-8580 (JP); KUJI Riku, Tokyo 174-8580 (JP)
(74) Representative: Gagel, Roland
(86) International application number: PCT/JP2024/032314
(87) International publication number: WO 2025/070030

(57) **Abstract**

This optical coherence tomography device includes an optical system and a control unit. The optical system is configured so as to allow an optical condition to be changed and includes an optical scanner, and is configured to scan an object under measurement with measurement light using an optical scanner and thereby acquire optical coherence tomography data for an object under measurement. The control unit controls the optical scanner so as to scan the object under measurement with the measurement light along a high-speed scan axis with reference to a reference position moving along a low-speed scan axis. The control unit changes an optical condition in synchronization with the period of a low-speed scan along the low-speed scan axis.

## Description

### [TECHNICAL FIELD]

This invention relates to an optical coherence tomography apparatus, a method of controlling the optical coherence tomography apparatus, and a program.

### [BACKGROUND ART]

Optical coherence tomography (OCT) apparatuses that are used to form images representing the surface morphology or the internal morphology of an object to be measured using light beam emitted from a laser light source or the like have been known. OCT performed in the OCT apparatuses is not invasive on the human body, and therefore is expected to be applied to the medical field or the biological field, in particular. For example, in the ophthalmic field, apparatuses for forming images of the fundus, the cornea, or the like have been in practical use. Such apparatuses using a method of OCT (OCT apparatuses) can be applied to observe various sites of an eye to be examined. In addition, because of the ability to acquire high-definition images, the OCT apparatuses are applied to the diagnosis of various eye diseases.

In case of imaging (photographing) the object to be measured at a wide angle, optimal optical conditions of an optical system for imaging differ for each photographed site (scan region), depending on a cross-sectional shape of the object to be measured. As a result, the image quality around the site of interest may not be sufficient, and the object to be measured may need to be re-imaged.

In contrast, the imaging range (measurement range, depth range) in the depth direction of OCT may be increased. However, the imaging range in the depth direction of OCT is limited. In order to increase the imaging range in the depth direction, the cost of a detection system becomes higher and the optical system becomes larger.

For example, Patent Document 1 discloses a method of performing a pre-scan, and of adjusting the optical path length of the reference light so that the entire tomographic image of the site to be observed is arranged at an optimal position in the B-scan image based on the A-scan images at a plurality of positions in a scan direction of B-scan acquired in advance.

### [PRIOR ART DOCUMENTS]

### [PATENT DOCUMENT]

[Patent Document 1] Japanese Unexamined Patent Publication No. 2015-16151

### [SUMMARY OF THE INVENTION]

### [PROBLEMS TO BE SOLVED BY THE INVENTION]

However, in Patent Document 1, the pre-scan has to be performed before the main scan, which increases the imaging time (measurement time). In case that the object to be measured is living eye, a problem of increased burden on the examinee arises.

The present invention has been made in view of such circumstances, and one of objects of the present invention is to provide a new technique for acquiring images of an object to be measured with good image quality in case of imaging the object to be measured at a wide angle.

### [MEANS OF SOLVING THE PROBLEMS]

One aspect of some embodiments is an optical coherence tomography apparatus including: an optical system configured to allow an optical condition to be changed, including an optical scanner, and configured to acquire optical coherence tomography data of an object to be measured by scanning the object to be measured with measurement light using the optical scanner; and a controller configured to control the optical scanner so as to scan the object to be measured with the measurement light along a fast scan axis with reference to a reference position moved along a slow scan axis, wherein the controller is configured to change the optical condition in synchronization with a period of the slow scan along the slow scan axis.

Another aspect of some embodiments is a method of controlling an optical coherence tomography apparatus including an optical system configured to allow an optical condition to be changed, including an optical scanner, and configured to acquire optical coherence tomography data of an object to be measured by scanning the object to be measured with measurement light using the optical scanner. The method of controlling the optical coherence tomography apparatus includes: a control step of controlling the optical scanner so as to scan the object to be measured with the measurement light along a fast scan axis with reference to a reference position moved along a slow scan axis; and an optical condition changing step of changing the optical condition in synchronization with a period of the slow scan along the slow scan axis.

Still another aspect of some embodiments is a program of causing a computer to execute each step of method of controlling the optical coherence tomography apparatus described above.

Still another aspect of the embodiments is a computer program product including the computer program(s)/instruction(s). The computer program product realizes each step in the method of controlling the optical coherence tomography apparatus described above, when the computer program(s)/instruction(s) is/are executed by the processor.

Still another aspect of the embodiments is a computer readable storage medium (recording medium) in which the computer program(s)/instruction(s) is/are stored. The computer readable recording medium realizes each step of the method of controlling the optical coherence tomography apparatus according to the embodiments, when the computer program(s)/instruction(s) is/are executed by the processor.

### [EFFECTS OF THE INVENTION]

According to embodiments according to the present invention, a new technique for acquiring images of an object to be measured with good image quality in case of imaging the object to be measured at a wide angle can be provided.

### [BRIEF EXPLANATION OF THE DRAWINGS]

[FIG. 1] FIG. 1 is a schematic diagram illustrating an example of a configuration of an optical system of an ophthalmic apparatus according to a first embodiment.
[FIG. 2] FIG. 2 is a schematic diagram illustrating an example of a configuration of an optical system of the ophthalmic apparatus according to the first embodiment.
[FIG. 3] FIG. 3 is a schematic diagram illustrating an example of a configuration of an optical system of the ophthalmic apparatus according to the first embodiment.
[FIG. 4] FIG. 4 is a schematic diagram illustrating an example of a configuration of a processing system of the ophthalmic apparatus according to the first embodiment.
[FIG. 5] FIG. 5 is a schematic diagram illustrating an example of a configuration of a processing system of the ophthalmic apparatus according to the first embodiment.
[FIG. 6] FIG. 6 is an explanatory diagram of an operation of the ophthalmic apparatus according to the first embodiment.
[FIG. 7] FIG. 7 is an explanatory diagram of an operation of the ophthalmic apparatus according to the first embodiment.
[FIG. 8] FIG. 8 is an explanatory diagram of an operation of the ophthalmic apparatus according to the first embodiment.
[FIG. 9] FIG. 9 is an explanatory diagram of an operation of the ophthalmic apparatus according to the first embodiment.
[FIG. 10] FIG. 10 is an explanatory diagram of an operation of the ophthalmic apparatus according to the first embodiment.
[FIG. 11] FIG. 11 is an explanatory diagram of an operation of the ophthalmic apparatus according to the first embodiment.
[FIG. 12A] FIG. 12A is a flowchart illustrating an example of an operation of the ophthalmic apparatus according to the first embodiment.
[FIG. 12B] FIG. 12B is a flowchart illustrating an example of an operation of the ophthalmic apparatus according to the first embodiment.
[FIG. 13A] FIG. 13A is a flowchart illustrating an example of an operation of the ophthalmic apparatus according to the first embodiment.
[FIG. 13B] FIG. 13B is a flowchart illustrating an example of an operation of the ophthalmic apparatus according to the first embodiment.
[FIG. 14] FIG. 14 is a schematic diagram illustrating an example of a configuration of an optical system of the ophthalmic apparatus according to a second embodiment.
[FIG. 15] FIG. 15 is a schematic diagram illustrating an example of a configuration of a processing system of the ophthalmic apparatus according to the second embodiment.
[FIG. 16] FIG. 16 is an explanatory diagram of an operation of the ophthalmic apparatus according to a first modification example of the first embodiment or the second embodiment.
[FIG. 17] FIG. 17 is an explanatory diagram of an operation of the ophthalmic apparatus according to a second modification example of the first embodiment or the second embodiment.
[FIG. 18] FIG. 18 is an explanatory diagram of an operation of the ophthalmic apparatus according to a third modification example of the first embodiment or the second embodiment.

### [MODES FOR CARRYING OUT THE INVENTION]

Referring now to the drawings, exemplary embodiments of an optical coherence tomography apparatus, a method of controlling the optical coherence tomography apparatus, and a program according to the present invention are described below. Any of the contents of the documents cited in the present specification and arbitrary known techniques may be applied to the embodiments below.

An optical coherence tomography (OCT) apparatus according to the embodiments includes an optical system configured to allow an optical condition of the optical system to be changed. The optical system includes an optical scanner, and is configured to acquire optical coherence tomography data of an object to be measured by scanning the object to be measured with measurement light used for OCT using the optical scanner. The OCT apparatus further includes a controller configured to control the optical scanner so as to scan the object to be measured with the measurement light using a single scan that combines a slow (low-speed) scan along a slow scan axis and a fast (high-speed) scan along a fast scan axis. Specifically, the controller is configured to control the optical scanner so as to scan the object to be measured with the measurement light along the fast scan axis with reference to a reference position of the fast scan moved along a slow scan axis. Further, the controller is configured to change the optical condition(s) of the optical system in synchronization with a period of the slow scan along the slow scan axis during scanning (imaging, measuring).

In case that a first fast scan is performed during the single scan that combines the slow scan and the fast scan, such an OCT apparatus can change the optical condition(s) of the optical system in synchronization with the period of the slow scan based on the OCT data acquired by performing a second fast scan that has been performed prior to the first fast scan. In some embodiments, the optical condition(s) of the optical system is/are fixed (kept) during the fast scan.

In some embodiments, the controller is configured to change the optical condition(s) described above, based on the OCT data or an OCT image. Here, the OCT data is acquired by performing the fast scan (or A-scan) that has been performed prior to the fast scan (or A-scan) in which the optical condition(s) of the optical system is/are changed during scanning, and the OCT image is formed based on the OCT data. Examples of the fast scan that has been performed prior to the fast scan (or A-scan) in which the optical condition(s) of the optical system is/are changed include a fast scan that has been performed immediately preceding the first fast scan in which the optical condition(s) of the optical system is/are changed, and a fast scan that has been performed before two or more scans prior to the first fast scan. In other words, the OCT apparatus substantially estimates the cross-sectional structure of the object to be measured based on the OCT data or the OCT image, which is acquired by performing the fast scan that has been performed in the past at a first fast scan position (region), and changes the optical condition(s) of the optical system in synchronization with the period of the slow scan based on the estimated cross-sectional structure. The OCT apparatus performs the fast scan at a second fast scan position, which is moved along the slow scan axis, different from the first fast scan position.

In some embodiments, the single scan is a scan that combines one slow scan (slow scan may be one or more slow scans) and two or more fast scans, the slow scan(s) and fast scans being performed from a predetermined scan start timing to a predetermined scan end timing. In some embodiments, the single scan is one scan that is performed from a predetermined scan start position to a predetermined scan end position.

In some embodiments, the OCT apparatus changes the optical condition(s) of the optical system for each scan region in synchronization with the period of the slow scan along the slow scan axis during scanning, for each of a plurality of scan regions set to the object to be measured. For example, the OCT apparatus substantially estimates the cross-sectional structure of the object to be measured based on the OCT data or the OCT image, which is acquired by the fast scan that has been performed in the past, and changes the optical condition(s) of the optical system in synchronization with the period of the slow scan based on the estimated cross-sectional structure, for each scan region.

Examples of the optical condition of the optical system include a light quantity of the measurement light used for OCT, a beam diameter of the measurement light used for OCT, a focusing position of the measurement light used for OCT, a polarization component of the measurement light, an aberration correction component, an optical path length difference between the measurement light and reference light, and a position of the optical system relative to the object to be measured.

The slow scan may be a scan defined in any one-dimensional or two-dimensional scan mode. The fast scan may be a scan defined in any one-dimensional or two-dimensional scan mode. Examples of such slow scan or fast scan include a line scan, a cross scan, a circle scan, a radial scan, a concentric scan, a multiline cross scan, a helical scan (spiral scan),and a Lissajous scan. Further, the examples of the scan that combines the slow scan and the fast scan include a three-dimensional scan, such as an ammonite scan. The ammonite scan is performed by combining a slow scan, in which the slow scan axis is a spiral scan axis, and a fast scan, in which the fast scan axis is a circular scan axis.

Thereby, in case of imaging the object to be measured at a wide angle, an image of the object to be measured can be acquired with good image quality without performing pre-scan.

In some embodiments, the optical system includes one or more optical elements, and is configured to allow at least one of the focusing position of the measurement light used for OCT, the polarization component of the measurement light, the aberration correction component, or the optical path length difference between the measurement light and the reference light to be changed by controlling the one or more optical elements.

In some embodiments, the optical system includes a mechanism that moves the optical system or the one or more optical elements, and is configured to allow any of the focusing position of the measurement light used for OCT, the polarization component of the measurement light, the aberration correction component, and the optical path length difference between the measurement light and the reference light to be changed by controlling the mechanism.

In some embodiments, the OCT apparatus includes a movement mechanism that moves the optical system relative to the object to be measured, and is configured to allow the position of the optical system relative to the object to be measured to be changed by controlling the movement mechanism.

The focusing position is changed by the focusing position changing member as an optical element. Examples of the focusing position changing member include a lens that is movable along an optical axis, a liquid crystal lens, and an Alvarez lens.

The polarization component is changed by a polarization component changing member as an optical element. Examples of the polarization component include a polarization controller. In case of splitting light from a light source into the measurement light and the reference light, that are used for OCT, the polarization controller may change the polarization component of the light from the light source, or the polarization component of the measurement light.

The aberration correction component is changed by an aberration correction device as an optical element. Examples of the aberration correction device include a variable cross cylinder (hereinafter referred to as VCC) lens, a liquid crystal lens, a wavefront aberration correction optical system including a deformable mirror, and an Alvarez lens.

The optical path length difference is changed by an optical path length changing member as an optical element or a mechanism. Examples of the optical path length changing member include a member that changes at least one of the optical path length of the measurement light or the optical path length of the reference light.

The position of the optical system relative to the object to be measured is changed by a mechanism that changes at least one of the object to be measured or the optical system. Examples of the mechanism include a movement mechanism that changes a relative position between the object to be measured and a holding member that holds the optical system.

In some embodiments, the optical system includes an interference optical system and an optical path length difference changing member. The interference optical system is configured to split light from a light source into the measurement light and reference light, to project the measurement light having traveled through a measurement optical path, on which one or more optical elements are arranged, onto the object to be measured, and to detect interference light between returning light from the object to be measured and the reference light having traveled through a reference optical path. The optical path length difference changing member is configured to change a difference between an optical path length of the measurement light and an optical path length of the reference light. The controller is configured to change the optical condition(s) of the optical system by controlling at least one of the one or more optical elements or the optical path length difference changing member.

In some embodiments, the OCT apparatus includes an image forming unit configured to form an image of the object to be measured based on the OCT data, and an image quality evaluation value calculator configured to calculate an evaluation value of an image quality of the image of the object to be measured. The controller is configured to control the one or more optical elements described above based on the calculated evaluation value.

For example, the controller is configured to control the one or more optical elements described above based on the calculated evaluation value so that the image of the object to be measured have image quality equal to or greater than a predetermined image quality level. Examples of the image quality equal to or greater than a predetermined image quality level include image quality in which the evaluation value of image quality for the entire image is maximized, image quality in which the evaluation value of image quality for the entire image is equal to or greater than a predetermined threshold value level, image quality in which a statistical value of the evaluation values, each of which is calculated for each scan region, of a plurality of images is maximized, and image quality in which a statistical value of the evaluation values, each of which is calculated for each scan region, of a plurality of images is equal to or greater than a predetermined threshold value level. Here, examples of the statistical value include a maximum value, a minimum value, a median, an average value, a mode, a range, a variance, a standard deviation, a weighted average value where a weighting factor is larger the closer to the site of interest (optical axis of the optical system), or a value of a predetermined evaluation equation using any of the statistical values described above.

In some embodiments, the OCT apparatus includes a movement mechanism configured to relatively move the optical system relative to the object to be measured. The controller is configured to change the optical condition(s) of the optical system by controlling the movement mechanism.

In some embodiments, the OCT apparatus includes an image forming unit. The controller is configured to control an optical path length difference changing member based on the position of the object be measured in the image.

In some embodiments, the scan region includes a central region including an optical axis of the optical system in the object be measured, and one or more peripheral regions around the central region. For example, the one or more peripheral regions includes one or more intermediate regions that are arranged outside the central region and surround the central region, and the peripheral region that is arranged outside the outermost intermediate region among the one or more intermediate regions and surrounds the outermost intermediate region.

Thereby, the image of the object to be measured can be acquired with good image quality, even when the inclination of the cross-section differs between the central region and the one or more peripheral regions of the object to be measured.

A method of controlling the OCT apparatus according to the embodiments is a method for controlling the OCT apparatus according to the embodiments. A method of controlling the OCT apparatus according to the embodiments includes one or more steps performed by the OCT apparatus described above. A program (computer program)/instruction according to the embodiments causes a computer to execute each step in the method of controlling the OCT apparatus according to the embodiments. A computer program product according to the embodiments includes the computer program(s)/instruction(s). The computer program product realizes each step of the method of controlling the OCT apparatus according to the embodiments, when the computer program(s)/instruction(s) are executed by the processor. A recording medium (storage medium) according to the embodiments is any computer readable non-transitory recording medium (storage medium) on which the program according to the embodiments is recorded. The computer readable recording medium according to the embodiments stores the computer program(s)/instruction(s). The computer readable recording medium realizes each step of the method of controlling the OCT apparatus according to the embodiments, when the computer program(s)/instruction(s) are executed by the processor. The recording medium may be an electronic medium using magnetism, light, magneto-optical, semiconductor, or the like. Typically, the recording medium is a magnetic tape, a magnetic disk, an optical disk, a magneto-optical disk, a flash memory, a solid state drive, or the like. Further, the program may be transmitted and received through a network such as the Internet, LAN, etc.

Hereinafter, an ophthalmic apparatus in which the object to be measured is a living eye will be described as an example of the OCT apparatus according to the embodiments. However, the embodiments can also be applied to OCT apparatus where the object to be measured is other than the living eye.

The ophthalmic apparatus according to the embodiments can perform OCT on an arbitrary site of the eye to be examined, such as a fundus, or an anterior segment, for example. In this specification, an image acquired using OCT may be collectively referred to as an "OCT image". Further, the measurement operation for forming OCT images may be referred to as OCT measurement (main measurement, provisional measurement).

Hereinafter, in the embodiments, the case of using the swept source type OCT method in the measurement and the imaging (photographing) using OCT will be described. However, the configuration according to the embodiments can also be applied to an ophthalmic apparatus using other type of OCT (for example, spectral domain type OCT or time domain OCT).

Furthermore, hereinafter, a case in which the evaluation value of image quality is higher the better the image quality is, and is lower the worse the image quality is, will be described. However, the embodiments can be applied to a case in which the evaluation value of image quality is lower the better the image quality is, and is higher the worse the image quality is.

### <First embodiment>

### [Configuration]

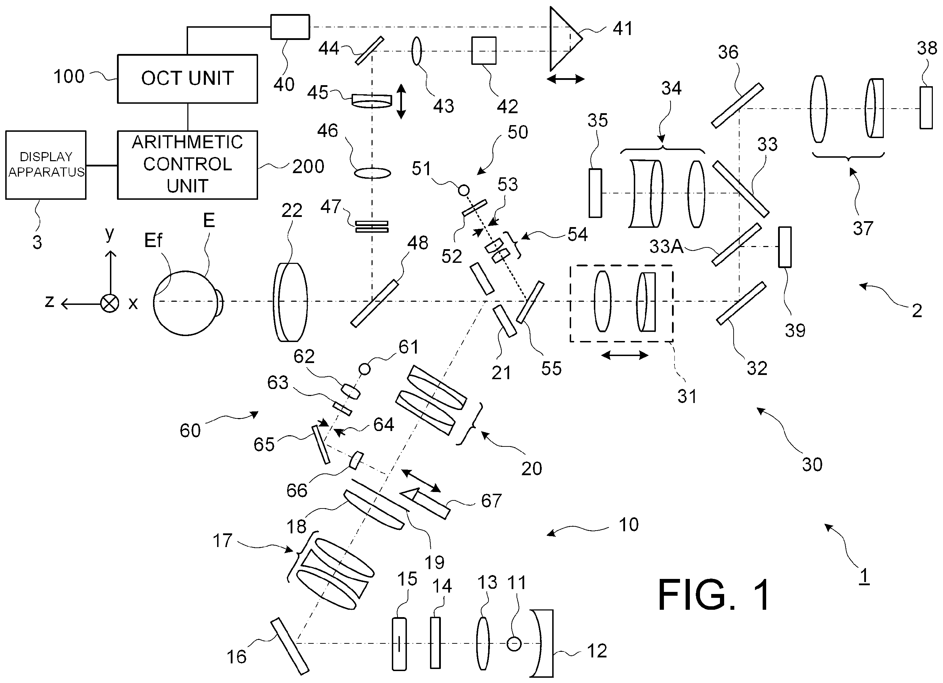

As shown in FIG. 1, FIG. 2, and FIG. 3, an ophthalmic apparatus 1 according to a first embodiment includes a fundus camera unit 2, an OCT unit 100, and an arithmetic control unit 200. The fundus camera unit 2 has substantially the same optical system as the conventional fundus camera. The OCT unit 100 is provided with an optical system for obtaining OCT images (for example, tomographic images) of the fundus (or the anterior segment). The arithmetic control unit 200 is provided a computer(s) that executes various kinds of arithmetic processing, control processing, and the like.

### [Fundus camera unit 2]

The fundus camera unit 2 illustrated in FIG. 1 is provided with an optical system for acquiring two-dimensional images (fundus images) representing the surface morphology of a fundus Ef of an eye E to be examined. Examples of the fundus images include observation images and photographic images. The observation image is, for example, a monochrome moving image formed at a predetermined frame rate using near-infrared light. The photographic image may be, for example, a color image captured by flashing visible light, or a monochrome still image using near-infrared light or visible light as illumination light. The fundus camera unit 2 may be configured to be capable of acquiring other types of images such as fluorescein angiograms, indocyanine green angiograms, and autofluorescent angiograms.

The fundus camera unit 2 is provided with a jaw holder and a forehead rest for supporting the face of a subject (examinee). Further, the fundus camera unit 2 is provided with an illumination optical system 10 and an imaging optical system 30. The illumination optical system 10 irradiates illumination light onto the fundus Ef. The imaging optical system 30 guides fundus reflected light of this illumination light to an imaging device (i.e., the CCD image sensor 35 or 38). Each of the CCD image sensors 35 and 38 is sometimes simply referred to as a "CCD". Further, the imaging optical system 30 guides measurement light coming from the OCT unit 100 to the fundus Ef, and guides the measurement light via the fundus Ef to the OCT unit 100.

An observation light source 11 in the illumination optical system 10 includes, for example, a halogen lamp. Light (observation illumination light) emitted from the observation light source 11 is reflected by a reflective mirror 12 having a curved reflective surface, travels through a condenser lens 13, and becomes near-infrared light after passing through a visible cut filter 14. Further, the observation illumination light is once converged near an imaging light source 15, is reflected by a mirror 16, and passes through relay lenses 17 and 18, a diaphragm 19, and a relay lens 20. Then, the observation illumination light is reflected on the peripheral part (the surrounding area of the hole part) of the perforated mirror 21, is transmitted through a dichroic mirror 48, and refracted by the objective lens 22, thereby illuminating the fundus Ef. It should be noted that an LED (light emitting diode) may be used as the observation light source.

Fundus reflected light of the observation illumination light is refracted by the objective lens 22, is transmitted through the dichroic mirror 48, passes through the hole part formed in the center area of the perforated mirror 21, is transmitted through a dichroic mirror 55, travels through a focusing lens 31, and is reflected by a mirror 32. Further, this fundus reflected light is transmitted through a half mirror 33A, is reflected by a dichroic mirror 33, and forms an image on the light receiving surface of the CCD image sensor 35 by a condenser lens 34. The CCD image sensor 35 detects the fundus reflected light at a predetermined frame rate, for example. An image (observation image) based on the fundus reflected light detected by the CCD image sensor 35 is displayed on a display apparatus 3. It should be noted that when the imaging optical system 30 is focused on the anterior segment, an observation image of the anterior segment of the eye E to be examined is displayed.

The imaging light source 15 includes, for example, a xenon lamp. Light (imaging illumination light) emitted from the imaging light source 15 is irradiated onto the fundus Ef through the same route as that of the observation illumination light. The fundus reflected light of the imaging illumination light is guided to the dichroic mirror 33 via the same route as that of the observation illumination light, is transmitted through the dichroic mirror 33, is reflected by a mirror 36, and forms an image on the light receiving surface of the CCD image sensor 38 by a condenser lens 37. The display apparatus 3 displays an image (photographic image) obtained based on the fundus reflected light detected by the CCD image sensor 38. It should be noted that the display apparatus 3 for displaying the observation image and the display apparatus 3 for displaying the photographic image may be the same or different. Besides, when similar imaging is performed by illuminating the eye E to be examined with infrared light, an infrared photographic image is displayed. It is also possible to use an LED as the imaging light source.

A liquid crystal display (LCD) 39 displays a fixation target and a visual target used for visual acuity measurement. The fixation target is a visual target for fixating the eye E to be examined, and is used when performing fundus imaging (photography) and OCT measurement.

Part of light emitted from the LCD 39 is reflected by the half mirror 33A, is reflected by the mirror 32, travels through the focusing lens 31 and the dichroic mirror 55, and passes through the hole part of the perforated mirror 21. The light having passed through the hole part of the perforated mirror 21 is transmitted through the dichroic mirror 48, and is refracted by the objective lens 22, thereby being projected onto the fundus Ef.

By changing the display position of the fixation target on the screen of the LCD 39, the fixation position of the eye E to be examined can be changed. Examples of the fixation position of the eye E to be examined include a position for acquiring an image centered at a macular region of the fundus Ef, a position for acquiring an image centered at an optic disc, and a position for acquiring an image centered at the fundus center between the macular region and the optic disc. Further, the display position of the fixation target may be changed to any desired position.

In addition, as with a conventional fundus camera, the fundus camera unit 2 is provided with an alignment optical system 50 and a focus optical system 60. The alignment optical system 50 generates an indicator (referred to as an alignment indicator) for the position matching (i.e., the alignment) of the optical system with respect to the eye E to be examined. The focus optical system 60 generates a target (split indicator) for adjusting the focus with respect to the fundus Ef.

The light output from an LED 51 of the alignment optical system 50 (i.e., alignment light) travels through the diaphragms 52 and 53 and the relay lens 54, is reflected by the dichroic mirror 55, and passes through the hole part of the perforated mirror 21. The light having passed through the hole part of the perforated mirror 21 is transmitted through the dichroic mirror 48, and is projected onto the cornea of the eye E to be examined by the objective lens 22.

Cornea reflected light of the alignment light travels through the objective lens 22, the dichroic mirror 48 and the hole part described above. Part of the cornea reflected light is transmitted through the dichroic mirror 55, and passes through the imaging focusing lens 31, is reflected by the mirror 32, and is transmitted through the half mirror 33A. The cornea reflected light transmitted through the half mirror 33A is reflected by the dichroic mirror 33, and forms an image on the light receiving surface of the CCD image sensor 35 by the condenser lens 34. The light receiving image (i.e., alignment indicator image) captured by the CCD image sensor 35 is displayed on the display apparatus 3 together with the observation image. A user performs an alignment in the same manner as performed on a conventional fundus camera. Alternatively, alignment may be performed in such a way that the arithmetic control unit 200 analyzes the position of the alignment indicator and moves the optical system (automatic alignment).

To perform focus adjustment, a reflective surface of a reflection rod 67 is arranged in a slanted position on an optical path of the illumination optical system 10. The light output from a LED 61 in the focus optical system 60 (i.e., focus light) passes through a relay lens 62, is split into two light beams by a split indicator plate 63, passes through a two-hole diaphragm 64, and is reflected by a mirror 65. The focus light reflected by the mirror 65 is once converged on the reflective surface of the reflection rod 67 by the condenser lens 66, and is reflected by the reflective surface. Further, the focus light travels through the relay lens 20, is reflected by the perforated mirror 21, is transmitted through the dichroic mirror 48, and is refracted by the objective lens 22, thereby being projected onto the fundus Ef.

Fundus reflected light of the focus light passes through the same route as the cornea reflected light of the alignment light and is detected by the CCD image sensor 35. The display apparatus 3 displays the light receiving image (split indicator) captured by the CCD image sensor 35 together with the observation image. As in the conventional case, the arithmetic control unit 200 analyzes the position of the split indicator, and moves the focusing lens 31 and the focus optical system 60 for focusing (automatic focusing). Alternatively, the user may manually perform the focus adjustment while visually checking the split indicator.

The dichroic mirror 48 branches the optical path for OCT measurement from the optical path for fundus imaging (photography). The dichroic mirror 48 reflects light of wavelengths used for OCT measurement, and transmits light for fundus imaging. The optical path for OCT measurement is provided with, in order from the OCT unit 100 side, a collimator lens unit 40, an optical path length changing unit 41, an optical scanner 42, a collimator lens 43, a mirror 44, an OCT focusing lens 45. a field lens 46, and a VCC lens 47.

The optical path length changing unit 41 is configured to be capable of moving in a direction indicated by the arrow in FIG. 1, thereby changing the optical path length for OCT measurement. The change in the optical path length is used for the correction of the optical path length according to the axial length of the eye E to be examined, and/or for the adjustment of the interference state, or the like. The optical path length changing unit 41 includes, for example, a corner cube and a mechanism for moving the corner cube.

The optical scanner 42 is disposed at a position conjugate optically to a pupil of the eye E to be examined (pupil conjugate position) or near the position. The optical scanner 42 changes the traveling direction of light (measurement light) traveling along the optical path for OCT measurement. The optical scanner 42 can deflect the measurement light in a onedimensionally or two-dimensional manner, under the control from the arithmetic control unit 200 described below.

The optical scanner 42 includes a first galvanometer mirror, a second galvanometer mirror, and a mechanism for driving them independently, for example. The first galvanometer mirror deflects measurement light LS so as to scan the imaging site (fundus Ef or the anterior segment) in a horizontal direction (x-direction) orthogonal to an optical axis of the interference optical system. The second galvanometer mirror deflects the measurement light LS deflected by the first galvanometer mirror so as to scan the imaging site in a vertical direction (y direction) orthogonal to the optical axis of the interference optical system. Thereby, the imaging site can be scanned with the measurement light LS in any direction on the x-y plane.

For example, by controlling an orientation of the first galvanometer mirror and an orientation of the second galvanometer mirror included in the optical scanner 42 at the same time, the irradiated position of the measurement light can be moved along an arbitrary trajectory on the x-y plane. This allows to scan the imaging site according to a desired scan pattern.

The OCT focusing lens 45 is movable along the optical path of the measurement light LS (the optical axis of the interference optical system). The OCT focusing lens 45 moves along the optical path of the measurement light LS, under the control from the arithmetic control unit 200 described below.

In some embodiments, a liquid crystal lens or an Alvarez lens is provided instead of the OCT focusing lens 45. The liquid crystal lens or the Alvarez lens, as well as the OCT focusing lens 45, is controlled by the arithmetic control unit 200.

The VCC lens 47 is arranged on the optical path of the measurement light and changes at least one of the cylindrical power (astigmatic power) or the cylindrical axis angle (astigmatic axis angle). The VCC lens 47 has two cylindrical lenses (optical elements) positioned opposite each other, and is configured to change at least one of the cylindrical power or the cylindrical axis angle by changing at least one of the axial directions of the two cylindrical lenses. In the first embodiment, the two cylindrical lenses are configured to be capable of rotating independently so that the two axial directions are changed relative to each other. Further, the two cylindrical lenses are configured to be capable of rotating integrally while keeping the angle formed by the two axial directions.

The VCC lens 47 is disposed at a position conjugate optically to the pupil of the eye to be examined (pupil conjugate position) or near the position. In the first embodiment, since the optical scanner 42 is disposed at the position conjugate optically to the pupil of the eye to be examined, the VCC lens 47 is disposed near the position conjugate optically to the pupil of the eye to be examined.

For the purpose of correcting the astigmatic power of the eye E to be examined, even if the VCC lens 47 is disposed near the pupil conjugate position, it is reasonable to assume that a deviation of the arranged position of the VCC lens 47 relative to the pupil conjugate position has little effect on the cylindrical power or the cylindrical axis angle which are changed by the VCC lens 47.

When the VCC lens 47 is controlled based on the optometric data (objective measurement values or subjective inspection values) of the eye E to be examined, the optometric data are mainly measured values on the fovea of the eye E to be examined. However, it is reasonable to assume that the deviation of the arranged position of the VCC lens 47 relative to the pupil conjugate position has little effect on the cylindrical power or the cylindrical axis angle which are changed by the VCC lens 47. Therefore, even if the imaging site is different from the fovea, the VCC lens 47 may be positioned near the pupil conjugate position.

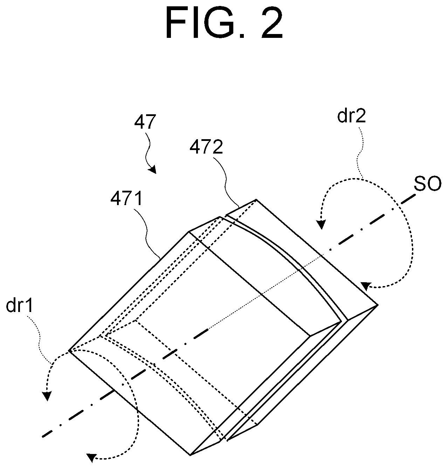

Such a VCC lens 47 is configured to include cylindrical lenses 471 and 472 with equal power and different signs from each other (focal lengths f0 and - f0), as shown in FIG. 2, for example. The cylindrical lens 471 (VCC1) has a convex surface (positive power), and is provided to be capable of rotating in a rotational direction dr1 around the optical path of measurement light LS (optical axis SO of the interference optical system). The cylindrical lens 472 (VCC2) has a concave surface (negative power), and is provided to be capable of rotating in a rotational direction dr2 around the optical axis SO. The cylindrical lenses 471 and 472 are driven by a driver such as a pulse motor, and are rotated independently around the optical axis SO. When the cylindrical lenses 471 and 472 are rotated in opposite directions to each other, the cylindrical power is changed, and when the cylindrical lenses 471 and 472 are rotated integrally in the same direction, the cylindrical axis angle is changed.

For example, the cylindrical lenses 471 and 472 are rotated in opposite directions to each other from a state in which the cylindrical axis angle of the cylindrical lenses 471 and 472 are tilted at a predetermined angle (e.g., 45 degrees) with respect to the optical axis SO. Thereby, arbitrary cylindrical power can be generated. Further, by rotating the cylindrical lenses 471 and 472 integrally in the same direction, arbitrary cylindrical axis angle can be generated.

### [OCT unit 100]

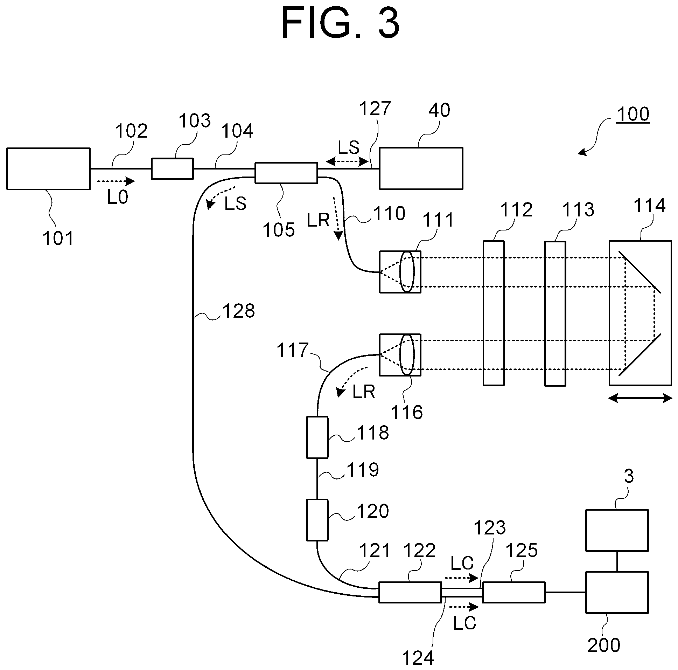

The configuration of the OCT unit 100 will be described with reference to FIG. 3. The OCT unit 100 is provided with an optical system for acquiring OCT images of the fundus Ef. The optical system has the same configuration as the conventional swept source type OCT apparatus. That is, the optical system includes an interference optical system configured to split light from a wavelength scanning type (wavelength sweeping type) light source into measurement light and reference light, to make the measurement light returned from the fundus Ef and the reference light having passed through a reference optical path interfere with each other to generate interference light, and to detect the interference light. The detection result (detection signal) of the interference light obtained by the interference optical system is a signal indicating the spectra of the interference light and is sent to the arithmetic control unit 200.

Like the general swept source type OCT apparatus, a light source unit 101 includes a wavelength scanning type (wavelength sweeping type) light source capable of scanning (sweeping) the wavelengths of emitted light. The light source unit 101 temporally changes the output wavelengths within the near-infrared wavelength bands that cannot be visually recognized with human eyes.

The light L0 emitted from the light source unit 101 is guided to a polarization controller 103 through an optical fiber 102, and a polarization state of the light L0 is adjusted. The polarization controller 103, for example, applies external stress to the looped optical fiber 102 to thereby adjust the polarization state of the light L0 guided through the optical fiber 102.

The light L0 whose polarization state has been adjusted by the polarization controller 103 is guided to a fiber coupler 105 through an optical fiber 104, and is split into the measurement light LS and the reference light LR.

The reference light LR is guided to the collimator 111 through the optical fiber 110 and becomes a parallel light beam. The reference light LR, which has become the parallel light beam, is guided to a corner cube 114 via an optical path length correction member 112 and a dispersion compensation member 113. The optical path length correction member 112 acts as a delay means for matching the optical path length (i.e., the optical distance) of the reference light LR and that of the measurement light LS. The dispersion compensation member 113 acts as a dispersion compensation means for matching the dispersion characteristic of the reference light LR and that of the measurement light LS.

The corner cube 114 reverses the traveling direction of the reference light LR that has become the parallel light beam by the collimator 111. The optical path of the reference light LR incident on the corner cube 114 and the optical path of the reference light LR emitted from the corner cube 114 are parallel to each other. Further, the corner cube 114 is movable in a direction along the incident light path and the emitting light path of the reference light LR. Through such movement, the optical path length of the reference light LR (i.e., the reference optical path) is varied.

The reference light LR that has traveled through the corner cube 114 passes through the dispersion compensation member 113 and the optical path length correction member 112, is converted from the parallel light beam to a convergent light beam by a collimator 116, and enters an optical fiber 117. The reference light LR that has entered the optical fiber 117 is guided to a polarization controller 118. With the polarization controller 118, the polarization state of the reference light LR is adjusted.

The polarization controller 118 has the same configuration as, for example, the polarization controller 103. The reference light LR whose polarization state has been adjusted by the polarization controller 118 is guided to an attenuator 120 through an optical fiber 119, and the light quantity of the reference light LR is adjusted under the control of the arithmetic control unit 200. The reference light LR whose light quantity has been adjusted by the attenuator 120 is guided to the fiber coupler 122 through the optical fiber 121.

The measurement light LS generated by the fiber coupler 105 is guided through an optical fiber 127 and is collimated into a parallel light beam by the collimator lens unit 40. The measurement light LS made into a parallel light beam reaches the dichroic mirror 48 via the optical path length changing unit 41, the optical scanner 42, the collimator lens 43, the mirror 44, the OCT focusing lens 45, the field lens 46, and the VCC lens 47. Subsequently, the measurement light LS is reflected by the dichroic mirror 48, is refracted by the objective lens 22, and is projected onto the fundus Ef. The measurement light LS is scattered and reflected (including reflection) at various depth positions of the fundus Ef. Back-scattered light of the measurement light LS from the fundus Ef reversely advances along the same path as the outward path, and is guided to the fiber coupler 105. Then, the back-scattered light passes through an optical fiber 128, and arrives at the fiber coupler 122.

The fiber coupler 122 combines (interferes) the measurement light LS incident through the optical fiber 128 and the reference light LR incident through the optical fiber 121 to generate interference light. The fiber coupler 122 generates a pair of interference light LC by splitting the interference light generated from the measurement light LS and the reference light LR at a predetermined splitting ratio (for example, 50 : 50). The pair of the interference light LC emitted from the fiber coupler 122 is guided to the detector 125 through the optical fibers 123 and 124, respectively.

The detector 125 is, for example, a balanced photodiode that includes a pair of photodetectors for respectively detecting the pair of interference light LC and outputs the difference between the pair of detection results obtained by the pair of photodetectors. The detector 125 sends the detection result (i.e., detection signal) to the arithmetic control unit 200. For example, the arithmetic control unit 200 performs the Fourier transform etc. on the spectral distribution based on the detection result obtained by the detector 125 for each series of wavelength scanning (i.e., for each A-line) to form the tomographic image as the OCT image. The arithmetic control unit 200 causes the formed image to be displayed on the display apparatus 3.

Although a Michelson interferometer is employed in the first embodiment, it is possible to employ any type of interferometer such as Mach-Zehnder-type as appropriate. In the present embodiment, in addition to the configuration shown in FIG. 3, the interference optical system may further include the collimator lens unit 40, the optical path length changing unit 41, the optical scanner 42, the collimator lens 43, the mirror 44, the OCT focusing lens 45, the field lens 46, and the VCC lens 47, which are shown in FIG. 1. This interference optical system is an example of the "interference optical system" according to the embodiments. The VCC lens 47 (and VCC driver 47A described below) is an example of the "aberration correction device (aberration correction member, astigmatism correction optical member)" according to the embodiments. The OCT focusing lens 45 (and OCT focusing driver 45A described below) is an example of the "focusing position changing member" according to the embodiments. At least one of the optical path length changing unit 41 or the corner cube 114 (and reference driver 114A described below) is an example of the "optical path length changing member" according to the embodiments. At least one of the polarization controller 103 or 118 is an example of the "polarization state changing member" according to the embodiments.

### [Arithmetic control unit 200]

The configuration of the arithmetic control unit 200 will be described.

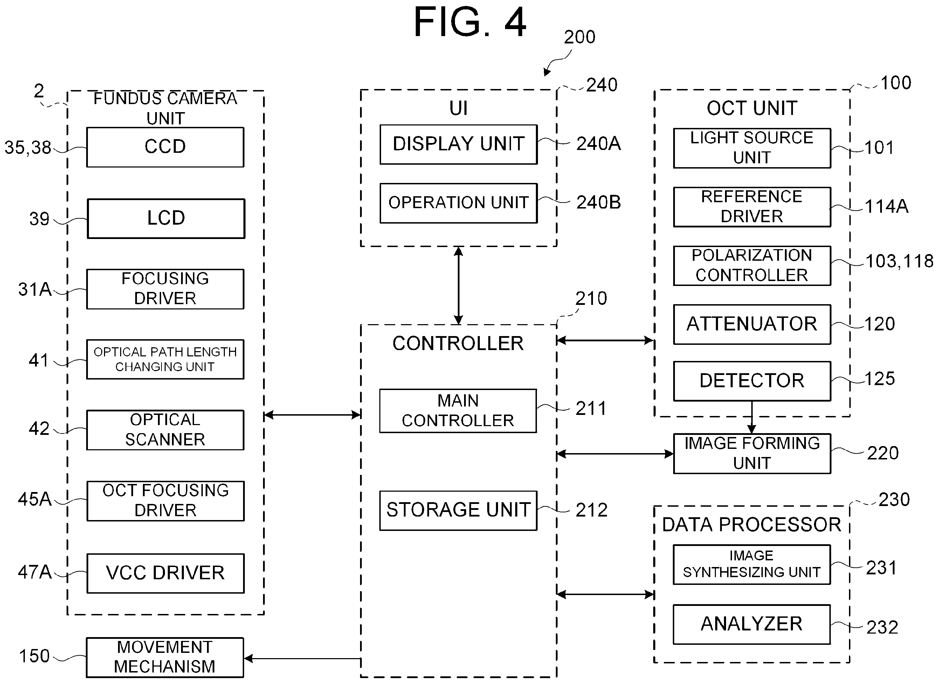

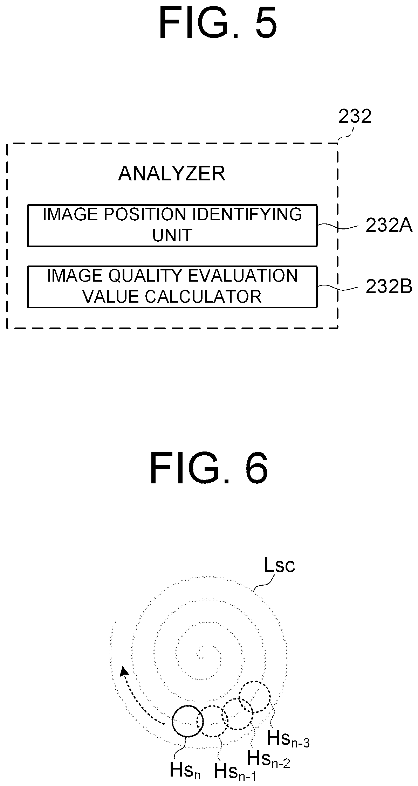

FIG. 4, and FIG. 5 show block diagrams of examples of a configuration of a processing system (control system) of the ophthalmic apparatus 1 according to the first embodiment. FIG. 5 shows a functional block diagram representing an example of a configuration of an analyzer 232 in FIG. 4. In FIG. 4, like reference numerals designate like parts as in FIG. 1, FIG. 2, and FIG. 3. The same description may not be repeated below.

The arithmetic control unit 200 analyzes the detection signals fed from the detector 125 to form an OCT image of the fundus Ef (or anterior segment). The arithmetic processing therefor is performed in the same manner as in the conventional swept-source-type OCT apparatus.

As shown in FIG. 4, the arithmetic control unit 200 includes a controller 210, and controls each part of the fundus camera unit 2, the display apparatus 3, and the OCT unit 100. For example, the arithmetic control unit 200 forms an OCT image, and causes the formed OCT image to be displayed on the display apparatus 3.

Examples of the control for the fundus camera unit 2 include the operation control for the observation light source 11, the imaging light source 15, the LEDs 51 and 61, the operation control for the CCD image sensors 35 and 38, the operation control for the LCD 39, the movement control for the focusing lens 31, the movement control for the OCT focusing lens 45, the movement control for the reflection rod 67, the operation control for the alignment optical system 50, the movement control for the focus optical system 60, the movement control for the optical path length changing unit 41, the driving control for the VCC lens 47, and the operation control for the optical scanner 42.

Examples of the control for the OCT unit 100 include the operation control for the light source unit 101, the movement control for the corner cube 114, the operation control for the detector 125, the operation control for the attenuator 120, and the operation controls for the polarization controllers 103 and 118.

Like conventional computers, the arithmetic control unit 200 includes a microprocessor, a RAM (random access memory), a ROM (read only memory), a hard disk drive, a communication interface, and the like. A storage device such as the hard disk drive stores a computer program for controlling the ophthalmic apparatus 1. The arithmetic control unit 200 may include various kinds of circuitry such as a circuit board for forming OCT images. In addition, the arithmetic control unit 200 may include an operation device (or an input device) such as a keyboard and a mouse, and a display device such as an LCD.

The processor includes, for example, a circuit(s) such as, for example, a CPU (central processing unit), a GPU (graphics processing unit), an ASIC (application specific integrated circuit), and a PLD (programmable logic device). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program. At least a part of the storage circuit or the storage apparatus may be included in the processor. Further, at least a part of the storage circuit or the storage apparatus may be provided outside of the processor. In some embodiments, the functions of the arithmetic control unit 200 are realized by one or more processors.

The fundus camera unit 2, the display apparatus 3, the OCT unit 100, and the arithmetic control unit 200 may be integrally provided (i.e., in a single housing), or they may be separately provided in two or more housings.

The controller 210 includes a main controller 211 and a storage unit 212.

### (Main controller 211)

The main controller 211 performs various controls by outputting control signals to each part of the ophthalmic apparatus 1 described above. In particular, the main controller 211 controls the CCD image sensors 35 and 38, the LCD 39, the focusing driver 31A, the optical path length changing unit 41, the optical scanner 42, the OCT focusing driver 45A, and the VCC driver 47A for the fundus camera unit 2. Further, the main controller 211 controls the light source unit 101, the reference driver 114A, the polarization controllers 103 and 118, the attenuator 120, and the detector 125 for the OCT unit 100.

The main controller 211 controls an exposure time (charge accumulation time), a sensitivity, a frame rate, or the like of the CCD image sensor 35 or 38. In some embodiments, the main controller 211 controls the CCD image sensor 35 or 38 so as to acquire images having the desired image quality.

The main controller 211 performs display control of fixation targets or visual targets for the visual acuity measurement, for the LCD 39. Thereby, the visual target presented to the eye E to be examined can be switched, or type of the visual targets can be changed. Further, the presentation position of the visual target to the eye E to be examined can be changed by changing the display position of the visual target on the screen of the LCD 39.

The focusing driver 31A moves the focusing lens 31 in the optical axis direction. The main controller 211 controls the focusing driver 31A so that the focusing lens 31 is positioned at a desired focusing position. As a result, the focusing position of the imaging optical system 30 is changed.

For example, the main controller 211 analyzes the position of the split indicator in the light receiving image (split indicator) obtained by the CCD image sensor 35, and controls the focusing driver 31A and the focus optical system 60. Alternatively, for example, the main controller 211 controls the focusing driver 31A and the focus optical system 60, according to the operations performed by the user on the operation unit 240B described below, while displaying a live image of the eye E to be examined on the display unit 240A described below.

The main controller 211 controls the optical path length changing unit 41 to change the optical path length of the measurement light LS. Thereby, the difference between the optical path length of the measurement light LS and the optical path length of the reference light LR is changed.

For example, the main controller 211 analyzes the detection result of the interference light LC obtained by OCT measurement (or the OCT image formed based on the detection result), and controls the optical path length changing unit 41 so that the measurement site is positioned at a desired depth position.

The main controller 211 is configured to control the optical scanner 42. The main controller 211 controls the optical scanner 42 so as to deflect the measurement light LS according to the deflection pattern corresponding to the scan mode set in advance.

Examples of scan mode includes a like this include a line scan, a cross scan, a circle scan, a radial scan, a concentric scan, a multiline cross scan, a helical scan (spiral scan), a Lissajous scan, and a three-dimensional scan (including a scan that combines a slow scan and a fast scan).

The line scan is a scan mode in which the measurement light LS is deflected so that the movement trajectory of the irradiated positions of the measurement light LS at the imaging site (measurement site) is line-shaped. The line direction of the movement trajectory can be changed (rotatable) around the optical axis on the x-y plane.

For example, the line scan includes a horizontal scan and a vertical scan. The horizontal scan is a scan mode in which measurement light LS is deflected so that the movement trajectory of the irradiated positions of the measurement light LS is in the horizontal direction (x-direction). The horizontal scan also includes a mode in which the measurement light LS is deflected along a plurality of scan lines that are arranged in the vertical direction (y-direction) and extend in the horizontal direction. In this mode, the intervals between the scan lines may be set as desired. Further, the intervals between the scan lines may be set sufficiently small to form a three-dimensional image. Such a scan mode is referred to as three-dimensional scan. These items for the horizontal scan mode may be applied to the vertical scan mode in similar ways.

The cross scan is a scan mode in which the measurement light LS is deflected so that the movement trajectory of the irradiated positions of the measurement light LS at the imaging site (measurement site) is cross-shaped. For example, the cross scan can be performed by executing two line scans whose line directions cross each other. The angle at which the two line scans intersect can be changed. In some embodiments, the scan lengths in the B-scan direction of the two line scans are identical. In some embodiments, the scan lengths in the B-scan direction of the two line scans are different.

The circle scan is a scan mode in which the measurement light LS is deflected so that the movement trajectory of the irradiated positions of the measurement light LS at the imaging site (measurement site) is, for example, in a circle around the optical axis SO. For example, in the circle scan, the measurement light LS is deflected so that the movement trajectory is a perfect circle, ellipse, or arc (part of a circumference).

The radial scan is a scan mode in which the measurement light LS is deflected so that the movement trajectory of the irradiated positions of the measurement light LS at the imaging site (measurement site) is radial around the optical axis SO, for example. In the radial scan mode, the irradiated positions of the measurement light LS are moved along a radial trajectory consisting of a plurality of straight lines arranged via a predetermined angle. The cross scan described above is one mode of the radial scan.

For example, in the radial scan, two or more line scans with B-scan directions different from each other are performed. In some embodiments, the scan lengths in the B-scan direction of the two or more line scans are identical. In some embodiments, the scan length in the B-scan direction of at least one of the two or more line scans is different from the other scan lengths.

The concentric scan is a scan mode in which the measurement light LS is deflected so that the movement trajectory of the irradiated positions of the measurement light LS at the imaging site (measured site) is concentric around the optical axis SO, for example. For example, in the concentric scan, the measurement light LS is deflected so that the movement trajectory of each circle is a perfect circle, ellipse, or arc (part of a circumference). In the concentric circle according to some embodiments, a plurality of circle scans with different diameters are performed in combination with each other. The circle scan is one mode of the concentric scan.

The multiline cross scan is a scan pattern in which a group of horizontal scan lines (for example, 5 lines) parallel to each other and a group of vertical scan lines (for example, 5 lines) parallel to each other are arranged so as to be perpendicular to each other in the vicinity of the center positions of the both groups of scan lines.

For example, in each group of scan lines in the multiline cross scan, two or more line scans are performed. In some embodiments, the scan lengths in the B-scan direction of the two or more line scans are identical. In some embodiments, the scan length in the B-scan direction of at least one of the two or more line scans is different from the other scan lengths.

The spiral scan (helical scan) is a scan mode in which the measurement light LS is deflected so that the movement trajectory of the irradiated positions of the measurement light LS at the imaging site (measurement site) is spiral around the optical axis SO, for example. In the spiral scan mode, the irradiated positions of the measurement light LS are moved along a spiral trajectory while the rotation radius is gradually reduced (or increased).

The Lissajous scan is a scan mode in which the measurement light LS is deflected so that the movement trajectory of the irradiated positions of the measurement light LS at the imaging site (measurement site) follows the Lissajous curve. The Lissajous scan is disclosed in Japanese Unexamined Patent Application Publication No. 2018-68578, for example.

Examples of the scan that combines the slow scan and the fast scan includes an ammonite scan (three-dimensional scan). The ammonite scan is a scan mode in which a scan reference position (scan center position) of the circle scan as a fast scan is moved along the scan pattern of the spiral scan as a slow scan. In other words, the circle scans are sequentially performed around each scan center position while moving the scan center position along the spiral scan pattern. As describe above, the slow scan may be a scan defined in any one-dimensional or two-dimensional scan mode, and the fast scan may be a scan defined in any one-dimensional or two-dimensional scan mode. Examples of the slow scan or the fast scan include a line scan, a cross scan, a circle scan, a radial scan, a concentric scan, a multiline cross scan, a helical scan (spiral scan), and a Lissajous scan.

By scanning the imaging site with the measurement light LS according to the deflection pattern corresponding to the scan mode as described above, the tomographic image as the OCT image in the plane stretched by the direction along the scan line (scan trajectory) and the fundus depth direction (z-direction) can be acquired.

The OCT focusing driver 45A moves the OCT focusing lens 45 along the optical axis of the measurement light LS. The main controller 211 controls the OCT focusing driver 45A so that the OCT focusing lens 45 is positioned at a desired focusing position. As a result, the focusing position of the measurement light LS is changed. The focusing position of the measurement light LS corresponds to the depth position (z position) of the beam waist of the measurement light LS.

For example, the main controller 211 controls the OCT focusing driver 45A based on a signal-to-noise ratio of the detection result of the interference light LC obtained by OCT measurement or evaluation value(s) (including statistical value of the evaluation values) corresponding to the image quality of the OCT image formed based on the detection result.

When the liquid crystal lens or the Alvarez lens is provided in place of the OCT focusing lens 45, the main controller 211 can control the liquid crystal lens or the Alvarez lens in the same way as it controls the OCT focusing driver 45A.

The VCC driver 47A rotates the cylindrical lenses 471 and 472 independently each other around the optical axis of the measurement light LS. Thereby, at least one of the cylindrical power or the cylindrical axis angle is changed.

For example, the main controller 211 controls the VCC driver 47A based on a signal-to-noise ratio of the detection result of the interference light LC obtained by OCT measurement or evaluation value(s) (including statistical value of the evaluation values) corresponding to the image quality of the OCT image formed based on the detection result. In some measurement light, the main controller 211 controls the VCC driver 47A based on a signal-to-noise ratio of the detection result of the interference light LC obtained by deflecting the measurement light LS according to the deflection pattern corresponding to the circle scan or evaluation value(s) (including statistical value of the evaluation values) corresponding to the image quality of the OCT image formed based on the detection result.

When a liquid crystal lens, a deformable mirror, or an Alvarez lens is provided in place of the VCC lens 47, the main controller 211 can control the liquid crystal lens, the deformable mirror, or the Alvarez lens in the same way as it controls the VCC driver 47A.

The main controller 211 controls the light source unit 101. The control for the light source unit 101 includes switching the light source on and off, controlling the intensity of the emitted light, changing the center frequency of the emitted light, changing the sweep speed of the emitted light, changing the sweep frequency, and changing the sweep wavelength range.

The reference driver 114A moves the corner cube 114 provided on the optical path of the reference light along this optical path. Thereby, the difference between the optical path length of the measurement light LS and the optical path length of the reference light LR is changed.

For example, the main controller 211 analyzes the detection result of the interference light LC obtained by OCT measurement (or the OCT image formed based on the detection result), and controls the reference driver 114A so that the measurement site is positioned at a desired depth position. In some embodiments, any one of the optical path length changing unit 41 and the reference driver 114A is provided.

The main controller 211 controls the polarization controllers 103 and 118. For example, the main controller 211 controls the polarization controllers 103 and 118 based on a signal-to-noise ratio of the detection result of the interference light LC obtained by OCT measurement or evaluation value(s) (including statistical value of the evaluation values) corresponding to the image quality of the OCT image formed based on the detection result.

The main controller 211 controls the attenuator 120. For example, the main controller 211 controls the attenuator 120 based on a signal-to-noise ratio of the detection result of the interference light LC obtained by OCT measurement or evaluation value(s) (including statistical value of the evaluation values) corresponding to the image quality of the OCT image formed based on the detection result.

The main controller 211 controls the detector 125. The control for the detector 125 includes the control for an exposure time (charge accumulation time), a sensitivity, a frame rate, or the like of the detector 125.

The movement mechanism 150 relatively moves the fundus camera unit 2 (OCT unit 100) relative to the eye E to be examined three-dimensionally. For example, the main controller 211 is capable of controlling the movement mechanism 150 to three-dimensionally move the optical system installed in the fundus camera unit 2. This control is used for alignment and/or tracking. Here, the tracking is to move the optical system of the apparatus according to the movement of the eye E to be examined. To perform tracking, alignment and focusing are performed in advance. The tracking is performed by moving the optical system of the apparatus in real time according to the position and orientation of the eye E to be examined based on the image obtained by photographing moving images of the eye E to be examined, thereby maintaining a suitable positional relationship in which alignment and focusing are adjusted.

In some embodiments, the main controller 211 corrects the position of scan range for OCT imaging in real time, based on tracking information obtained by performing tracking (tracking information obtained by tracking the optical system (interference optical system) with respect to a movement of the eye E to be examined). The main controller 211 can control the optical scanner 42 so as to scan the corrected scan range with the measurement light LS.

In some embodiments, the main controller 211 controls the optical system constituting the ophthalmic apparatus 1 based on the setting information stored in advance in the storage unit 212 to change the optical condition(s) (imaging condition, measurement condition) of the optical system. In this case, the main controller 211 can generate the setting information in advance based on the detection result of the interference light LC or the OCT image formed from the detection result. Here, the detection result of the interference light LC is acquired in the provisional measurement (provisional imaging) that has been performed before the main measurement (main imaging). Alternatively, the main controller 211 may generate the setting information in advance based on eye shape information (or aberration information of eye) statistically obtained from a plurality of eye information different from each other.

The main controller 211 causes the various information to be displayed on the display apparatus 3 (or display unit 240A described below), as a display controller. Examples of the information displayed on the display apparatus 3 include the results of imaging (observation image, OCT image (image of the eye to be examined formed based on the detection result of the interference light LC obtained by scanning with the measurement light LS), the measurement result (measured value), the evaluation value of the image quality of the image described below, the statistical value calculated based on the evaluation values, and evaluation metric information corresponding to the evaluation value. The main controller 211 can cause the evaluation value of the image quality of the OCT image, the statistical value, or the evaluation metric information to be displayed on the display apparatus 3 in association with the OCT image.

Further, the main controller 211 performs a process of writing data in the storage unit 212 and a process of reading out data from the storage unit 212.

### (Storage unit 212)

The storage unit 212 stores various types of data. Examples of the data stored in the storage unit 212 include image data of an OCT image, image data of a fundus image, and information on the eye to be examined. The information on the eye to be examined includes information on the examinee such as patient ID and name, and information on the eye to be examined such as identification information of the left/right eye. In addition, the storage unit 212 stores the optometric data obtained by an external device (e.g., a refractometer or subjective inspection apparatus) in advance, and various programs and data for operating the ophthalmic apparatus 1. The optometric data includes an astigmatic power of the eye to be examined, and an astigmatic axis angle of the eye to be examined. The optometric data may further include a spherical power of the eye to be examined. The optometric data may include at least one of a spherical power of the eye to be examined, an astigmatic power of the eye to be examined, or an astigmatic axis angle of the eye to be examined.

Further, the storage unit 212 can store the setting information described above. The setting information is information for setting the optical condition(s) of the optical system constituting the ophthalmic apparatus 1. More specifically, the setting information is information for setting the optical condition(s) of the optical system so that image quality of an image formed based on the OCT data, which is acquired using the optical system constituting the ophthalmic apparatus 1, becomes equal to or greater (higher) than a predetermined image quality level. Examples of the optical condition of the optical system constituting the ophthalmic apparatus 1 include a focusing position of the measurement light LS used for OCT, a polarization component of the measurement light LS, an aberration correction component, an optical path length difference between the measurement light LS and reference light LR, and a position of the optical system relative to the eye E to be examined.

At least part of the above data stored in the storage unit 212 may be stored in a storage unit provided outside the ophthalmic apparatus 1. For example, the ophthalmic apparatus 1 is connected so as to be capable of communicating with a sever apparatus having a function of storing at least part of the above data via a network such as an in-hospital LAN (Local Area Network). Here, the ophthalmic apparatus 1 and the server apparatus are connected via a WAN (Wide Area Network) such as the Internet. Further, the ophthalmic apparatus 1 and the server apparatus may be connected via a network that combines the LAN and the WAN.

### (Image forming unit 220)

An image forming unit 220 forms image data of the OCT image of the fundus Ef or the anterior segment based on the detection signal (interference signal, OCT data) from the detector 125. That is, the image forming unit 220 forms the image data of the eye E to be examined based on the detection result of the interference light LC obtained by the interference optical system. This processing includes processes such as noise removal (noise reduction), filter processing, and fast Fourier transform (FFT) in the same manner as the conventional swept source type OCT. The image data acquired in this manner is a data set including a group of image data formed by imaging the reflection intensity profiles of a plurality of A-lines. Here, the A-lines are the paths of the measurement light LS in the eye E to be examined.

In case of performing OCT scan for each scan region, the image forming unit 220 can form the image (OCT image) of the eye E to be examined for each scan region. In the present embodiment, the image forming unit 220 can form the OCT image of the eye E to be examined for each scan region, based on the OCT data acquired under the optical conditions different for each scan region.

In order to improve the image quality, it is possible to repeatedly perform scan with the same pattern a plurality of times to acquire a plurality of data sets, and to compose (i.e., average) the plurality of data sets.

The image forming unit 220 includes, for example, the circuitry described above. It should be noted that "image data" and an "image" based on the image data may not be distinguished from each other in the present specification. In addition, a site of the fundus Ef and an image of the site may not be distinguished from each other.

### (Data processor 230)

A data processor 230 performs various kinds of data processing (e.g., image processing) and various kinds of analysis processing on the detection result of the interference light LC or the image formed by the image forming unit 220. For example, the data processor 230 performs various correction processing, such as analysis processing of the signal-to-noise ratio of the interference signal, image brightness correction, dispersion correction, etc.

Further, the data processor 230 performs various kinds of image processing and various kinds of analysis processing on images (fundus images, anterior segment images, etc.) obtained by the fundus camera unit 2.

The data processor 230 performs known image processing such as interpolation processing for interpolating pixels between two-dimensional tomographic images to form image data of the three-dimensional image of the fundus Ef or the eye E to be examined. It should be noted that the image data of the three-dimensional image means image data in which the positions of pixels are defined in a three-dimensional coordinate system. Examples of the image data of the three-dimensional image include image data defined by voxels three-dimensionally arranged. Such image data is referred to as volume data or voxel data. When displaying an image based on volume data, the data processor 230 performs rendering processing (volume rendering, maximum intensity projection (MIP), etc.) on the volume data, thereby forming image data of a pseudo three-dimensional image viewed from a particular visual line. The pseudo three-dimensional image is displayed on the display device such as the display unit 240A.

Further, stack data of a plurality of tomographic images may be formed as the image data of the three-dimensional image. The stack data is image data obtained by three-dimensionally arranging tomographic images along a plurality of scan lines based on positional relationship of the scan lines. That is, the stack data is image data obtained by representing tomographic images, which are originally defined in their respective two-dimensional coordinate systems, by a single three-dimensional coordinate system. That is, the stack data is image data formed by embedding tomographic images into a single three-dimensional space.

The data processor 230 can build (form) the OCTA image (blood vessel emphasized image, angiogram) in which retinal blood vessels and choroidal blood vessels are emphasized (highlighted), based on data (for example, B-scan data, OCT data) acquired in time series by performing OCT scan. The OCTA image may be an image representing a cross-sectional structure or a front image.

In some embodiments, the data processor 230 compares the two OCT images acquired by repeatedly performing B-scans on the approximately same site of the eye E to be examined, and converts the pixel value of a change portion of the signal intensity into a pixel value corresponding to a change amount in the change portion to build the emphasized image (motion contrast image) in which the change portion is emphasized. Further, the data processor 230 can form the OCTA (angiography) image by extracting information of a predetermined thickness amount at a desired site from a plurality of built emphasized images and building as an en-face image.

The data processor 230 can perform position matching between the fundus image and the OCT image. When the fundus image and the OCT image are obtained in parallel, the position matching between the fundus image and the OCT image, which have been (almost) simultaneously obtained, can be performed using the optical axis of the imaging optical system 30 as a reference. Such position matching can be achieved since the optical system for the fundus image and that for the OCT image are coaxial. Besides, regardless of the timing of obtaining the fundus image and the OCT image, position matching between the fundus image and the OCT image can be achieved by registering the fundus image with an image obtained by projecting the OCT image onto the x-y plane. This position matching method can also be employed when the optical system for obtaining the fundus image and the optical system for OCT measurement are not coaxial. Further, when both the optical systems are not coaxial, if the relative positional relationship between these optical systems is known, the position matching can be performed with referring to the relative positional relationship in a manner similar to the case of coaxial optical systems.

Such a data processor 230 includes an image synthesizing unit 231 and an analyzer 232.

### (Image synthesizing unit 231)

The image synthesizing unit 231 can synthesize a plurality of images formed by the image forming unit 220 to generate a synthetic image. Specifically, the image synthesizing unit 231 performs position matching (alignment, registration) of the OCT images, which are formed by the image forming unit 220 for each scan region, to generate the synthetic image.

In some embodiments, the image synthesizing unit 231 performs position matching of the OCT images, which are formed for each scan region, based on the optical conditions different for each scan region or the setting information of the optical system corresponding to the optical condition(s) to generate the synthetic image.

In some embodiments, the image synthesizing unit 231 generates the synthetic image by performing position matching of the OCT images so that a correlation value in the overlapping region, where the peripheral regions of each of the OCT images formed for each scan region overlap, is maximized.

In some embodiments, the image synthesizing unit 231 generates the synthetic image by performing position matching of the OCT images based on a characteristic region such as a layer region, a vascular region, or disease site, which is depicted in each of the peripheral regions of the OCT images formed for each scan region.

### (Analyzer 232)

The analyzer 232 performs analysis processing for estimating a substantial cross-sectional structure of the eye E to be examined in addition to the analysis processing described above, based on the OCT data or the OCT image formed based on the OCT data. Here, the OCT data is acquired by performing OCT scan (fast scan) that has been performed in past. For example, the analyzer 232 performs analysis processing for estimating a substantial cross-sectional structure of the eye E to be examined for each scan region, based on the OCT data or the OCT image formed based on the OCT data. Here, the OCT data is acquired by performing OCT scan that has been performed for each scan region in past.

Such an analyzer 232 includes an image position identifying unit 232A and an image quality evaluation value calculator 232B.

### (Image position identifying unit 232A)

The image position identifying unit 232A identifies the substantial cross-sectional structure of the eye E to be examined based on a position in a depth direction of a site of interest (object to be measured (eye to be examined) in the broad sense) in the image. Examples of the site of interest include the fundus Ef (retina, retinal pigment epithelium layer), which has a curved cross-sectional shape.

In some embodiments, the image position identifying unit 232A identifies a position of the center of gravity in the depth direction (depth range) in the B-scan image (tomographic image) as the OCT image, and identifies the position in the depth direction of the site of interest in the B-scan image based on the identified position of the center of gravity in the depth direction. In this case, for example, the image position identifying unit 232A integrates (or averages integrated values) pixel values in the B-scan image in a direction (B-scan direction) perpendicular to the A-scan direction (depth direction). And, the image position identifying unit 232A identifies the position of the center of gravity in the depth direction based on the acquired integrated profile in the A-scan direction.

In some embodiments, the image position identifying unit 232A identifies a region having a predetermined shape as the region, where the site of interest is depicted, from the OCT image, and identifies the position in the depth direction of the identified region in the OCT image.

### (Image quality evaluation value calculator 232B)

The image quality evaluation value calculator 232B identifies the substantial cross-sectional structure of the eye E to be examined including the site of interest, based on the evaluation value (including the statistical value of the evaluation values) corresponding to the image quality (for example, signal-to-noise ratio) of the image. In the calculation processing of the evaluation value, the evaluation value is calculated by analyzing the detection result of the interference light, the OCT image, or a segmented image. Here, the OCT is formed by the image forming unit 220. The segmented image is obtained by segmenting (dividing) the OCT image. The segmented image may be an image obtained by segmenting the OCT image, corresponding to each of a plurality of scan regions obtained by segmenting the scan region. Alternatively, the segmented image may be an image obtained by segmenting the OCT image, corresponding to each of a preset plurality of scan regions.

The image quality evaluation value calculator 232B calculates arbitrary evaluation values quantitatively representing the image quality. Typically, the evaluation value is calculated so that the higher the quality of the image (image quality) is, the larger the value is. The calculation processing of the evaluation value performed by image quality evaluation value calculator 232B may be arbitrary processing. For example, the image quality evaluation value calculator 232B can perform processing using any known technology, such as the signal-to-noise ratio (SNR), the contrast-to-noise ratio (CNR), the root mean square (RMS) granularity, the Wiener spectrum, the modulation transfer function (MTF), the quality index (QI).

In the first example of the calculation processing of the evaluation value, the image quality evaluation value calculator 232B calculates a differential histogram of the image to be calculated, and calculates the evaluation value of the image quality using the maximum value of luminance values (differential values) with the number of pixels equal to or greater than a predetermined percentage in the entire image in the calculated differential histogram. Specifically, the image quality evaluation value calculator 232B calculates a difference between the maximum value of the differential values and the minimum value of the differential values at a predetermined threshold value (e.g., 20%) or higher as the evaluation value of the image quality (image quality evaluation value). In this case, the image quality evaluation value indicates a high value when the image quality is equal to or greater than a predetermined image quality level, and decreases as the image quality deviates from the predetermined image quality level.

In the second example of the calculation processing of the evaluation value, the data processor 230 (or analyzer 232) applies a predetermined analysis processing (e.g., segmentation processing) to an evaluation region set for an image corresponding to a predetermined site in the eye E to be examined. Thereby, the image quality evaluation value calculator 232B identifies an image regions (signal regions) corresponding to a desired site (tissue) and other image regions (non-signal regions). Next, the image quality evaluation value calculator 232B generates a histogram of luminance in the signal regions and a histogram of luminance in the non-signal regions. Subsequently, the image quality evaluation value calculator 232B calculates the evaluation value corresponding to the image quality from the degree of overlap of these two histograms. For example, the evaluation value is defined in the range of 0 to 100, such that when both histograms completely overlap, the evaluation value = 0, and when both histograms are completely separated, the evaluation value = 100. This evaluation operation may include, for example, normalization of two histograms, generation of a probability distribution function, and calculation of the evaluation value using a predetermined arithmetic formula.

Alternatively, the image quality evaluation value calculator 232B may calculate known parameters expressing the image quality, such as signal-to-noise ratio (SNR), spatial resolution, and contrast, for the image to be calculated. In this case, the image quality evaluation value indicates a high value when the image quality is equal to or greater than a predetermined image quality level, and decreases as the image quality deviates from the predetermined image quality level.

The image quality evaluation value calculator 232B calculates the evaluation value of the image quality of the entire OCT image, or the evaluation value of the image quality for each segmented image corresponding to each of the plurality of scan regions.

Further, the image quality evaluation value calculator 232B can calculate the statistical value of the evaluation values for a plurality of segmented images. Examples of the statistical value include a maximum value, a minimum value, a median, an average value, a mode, a range, a variance, a standard deviation, a weighted average value where a weighting factor is larger the closer to the site of interest (optical axis of the optical system), or a value of a predetermined evaluation equation using any of the statistical values described above.

Examples of the evaluation equation include an equation such that the higher the total value of the evaluation values of the segmented images and the smaller the variation of the evaluation values of the segmented images are, the larger the value VCCQ of the evaluation equation is. In this case, for example, the value VCCQ of the evaluation equation is a value obtained by dividing the sum of the evaluation values of the plurality of segmented images by (standard deviation of the plurality of evaluation values + 1).

The main controller 211 estimates the analysis result obtained by the analyzer 232 as the substantial cross-sectional structure of the eye E to be examined, and changes the optical condition(s) of the optical system in synchronization with the period of the slow scan based on the estimated cross-sectional structure. In some embodiments, the main controller 211 estimates the substantial cross-sectional structure of the eye E to be examined for each scan region, and changes the optical condition(s) of the optical system for each scan region in synchronization with the period of the slow scan based on the estimated cross-sectional structure.

The data processor 230 that functions described above includes, for example, a processor described above, a RAM, a ROM, a hard disk drive, a circuit board, and the like. Computer programs that cause a processor to execute the above functions are previously stored in a storage device such as a hard disk drive.

### (User interface 240)

The user interface 240 includes the display unit 240A and the operation unit 240B. The display unit 240A includes the display device of the arithmetic control unit 200 described above and/or the display apparatus 3. The operation unit 240B includes the operation device of the arithmetic control unit 200 described above. The operation unit 240B may include various kinds of buttons and keys provided on the housing of the ophthalmic apparatus 1, or provided outside the ophthalmic apparatus 1. For example, when the fundus camera unit 2 has a case similar to that of the conventional fundus camera, the operation unit 240B may include a joy stick, an operation panel, and the like provided to the case. Further, the display unit 240A may include various kinds of display devices, such as a touch panel placed on the housing of the fundus camera unit 2.

It should be noted that the display unit 240A and the operation unit 240B need not necessarily be formed as separate devices. For example, a device like a touch panel, which has a display function integrated with an operation function, can be used. In such cases, the operation unit 240B includes the touch panel and a computer program. The content of operation performed on the operation unit 240B is fed to the controller 210 as an electric signal. Moreover, operations and inputs of information may be performed using a graphical user interface (GUI) displayed on the display unit 240A and the operation unit 240B.

The eye E to be examined (fundus Ef, anterior segment) is an example of the "object to be measured" according to the embodiments. The optical system included in the OCT unit 100, the optical path length changing unit 41, the OCT focusing lens 45, and the VCC lens 47 (interference optical system) are an example of the "optical system" according to the embodiments. The OCT focusing lens 45, the corner cube 114, the optical path length changing unit 41, or the polarization controller 103 is an example of the "one or more optical elements" according to the embodiments. The focusing position of the measurement light LS, the polarization component of the measurement light LS, the aberration correction component, the optical path length difference between the measurement light LS and reference light LR, or the position of the optical system relative to the eye E to be examined is an example of the "optical condition of optical system" according to the embodiments. The ophthalmic apparatus 1 is an example of the "OCT apparatus" according to the embodiments. The optical path length changing unit 41, or, the corner cube 114 and reference driver 114A is an example of the "optical path length difference changing member" according to the embodiments. The OCT focusing lens 45 is an example of the "focusing lens" according to the embodiments. The VCC lens 47 is an example of the "aberration correction device" according to the embodiments. The display apparatus 3 or the display unit 240A is an example of the "display means" according to the embodiments.

Hereinafter, a case where the ophthalmic apparatus 1 according to the first embodiment performs ammonite scan on the eye E to be examined as an OCT scan will be described. However, the present embodiment also can be applied to cases where an OCT scan is performed on the eye E to be examined in a scan mode other than the ammonite scan.

FIG. 6 shows an explanatory diagram of the ammonite scan according to the first embodiment.

The ammonite scan according to the first embodiment is a scan mode in which one or more fast scans are sequentially performed with a scan pattern defined with reference to a moved scan center position, while the scan center position of the fast scan is moved along a scan pattern (scan trajectory) of the slow scan. In the ammonite scan according to the present embodiment, the fast scan is a circle scan and the slow scan is a spiral scan, where the circle scans, which are sequentially performed, are performed so that the circle scans intersect the scan pattern of the previous circle scan. In other words, the circle scan are sequentially performed so that the scan patterns of two adjacent circle scans intersect.

In FIG. 6, a scan pattern Lsc of the slow scan is schematically represented and fast scan patterns HSₙ (n is an integer of 4 or more), HSₙ₋₁, HSₙ₋₂, and HSₙ₋₃ are schematically represented.

The ammonite scan can perform scan on the eye E to be examined from a variety of scan directions with a simple control, by combining the slow scan and the fast scan, as described above. In particular, the region near the center of the ammonite scan (center position of slow scan) can be scanned at high density in a short period of time, and the farther away the scan position is from the center, the lower the density of the scan becomes in a longer period of time, which allows the scan of a wide region including the site of interest with a simple control.

According to the present embodiment, the eye E to be examined can be measured or imaged at a higher quality and wide angle regardless of the cross-sectional structure of the eye E to be examined, by scanning the eye E to be examined with the ammonite scan.

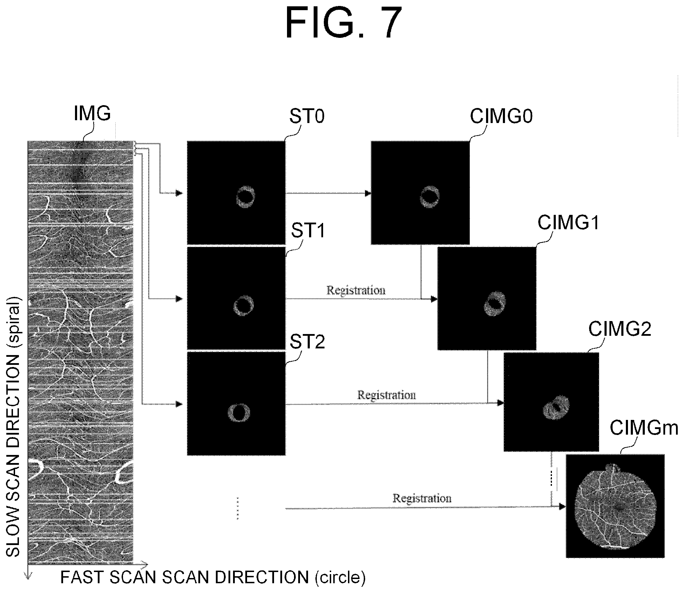

FIG. 7 schematically shows the OCT image formed based on the OCT data acquired by performing ammonite scan according to the first embodiment. In FIG. 7, a case where the OCTA image is formed will be described, for convenience of explanation. However, the same applies to the OCT image.

First, the main controller 211 sets the scan regions(s) of ammonite scan to the eye E to be examined. Examples of configuration parameter for setting the scan region of the ammonite scan include a scan start position of the slow scan (scan center position of slow scan), a scan end position of the slow scan, a scan speed of the slow scan, a change speed of scan radius from the scan center position of the slow scan, a scan speed of the fast scan, and a scan radius of the fast scan.

The main controller 211 control the optical scanner 42 and the OCT unit 100, etc. to repeat the movement of the scan center position of the fast scan along the scan pattern of the slow scan and two or more fast scans (circle scans) centered on the scan center positions after the movement. The main controller 211 controls the image forming unit 220 to form the OCT image (tomographic image, B-scan image) based on the acquired OCT data every time the circle scan is performed. Subsequently, the main controller 211 control the data processor 230 to sequentially form the OCTA images by comparing the two OCT images acquired by repeatedly performing circle scan (B-scan) on the approximately same site of the eye E to be examined. This allows to form the OCTA image IMG in which the OCTA images formed in the fast scan direction are arranged in the slow scan.

Next, the main controller 211 controls the data processor 230 to generate strip images ST0, ST1, ST2, ... by performing a coordinate transformation to the Cartesian coordinate system on each of a plurality of OCTA images formed in the fast scan direction. For example, the strip image ST0 is acquired by performing circle scan with the scan pattern HS₀ in FIG. 6. The strip image ST1 is acquired by performing circle scan with the scan pattern HS₁ intersecting the scan pattern HS₀. And, the strip image ST2 is acquired by performing circle scan with the scan pattern HS₂ intersecting the scan pattern HS₁. In this case, it is possible to thin out the OCTA images in which artifacts are generated due to the movement or blinking of the eye E to be examined during the circle scans, and to perform coordinate transformation merely on the OCTA images in which no artifacts are generated to acquire the strip images.

The main controller 211 controls the data processor 230 to generate synthetic images CIMG0, CIMG1, CIMG2, ..., CIMGm (m is an integer of 2 or more) by sequentially performing position matching (registration) of the strip images ST1, ST2, ..., using the strip image ST0 as the reference image. The data processor 230 performs position matching based on image (for example, vascular region in the OCTA image) within the scan region that overlaps in the two adjacent strip images.

Thereby, the synthetic image CIMGm in FIG. 7 can be acquired by performing ammonite scan in FIG. 6.

### [Operation Example]

The operation of the ophthalmic apparatus 1 according to the first embodiment will be described.

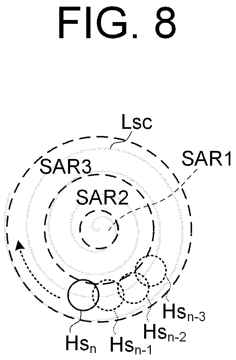

FIGs. 8 to 13B show examples of an operation of the ophthalmic apparatus 1 according to the first embodiment.

FIG. 8 schematically represents an example of a plurality of scan regions obtained by segmenting the scan region of the OCT scan performed in the ophthalmic apparatus 1 according to the first embodiment. In FIG. 8, the same parts as in FIG. 6 are shown by the same symbols and their descriptions will be omitted in an appropriate manner.

For example, the main controller 211 sets a central region SAR1 including the optical axis of the optical system in the eye E to be examined, an intermediate region SAR2 around the central region SAR1, and a peripheral region SAR3 around the intermediate region SAR2, as shown in FIG. 8. The intermediate region SAR2 is arranged outside the central region SAR1, and surrounds the central region SAR1. In FIG. 8, the intermediate region SAR2 may be two or more regions arranged in concentric circles. The peripheral region SAR3 is arranged outside the outermost intermediate region among the one or more intermediate regions including the central region SAR1, and is a region that surrounds the outermost intermediate region.

The main controller 211 can change the optical condition(s) of the optical system for each region shown in FIG. 8. Thereby, in case of imaging the object to be measured at a wide angle, an image of the eye E to be examined can be acquired with good image quality without performing pre-scan, while minimizing changes in the image due to changes in the optical condition(s) during scanning.

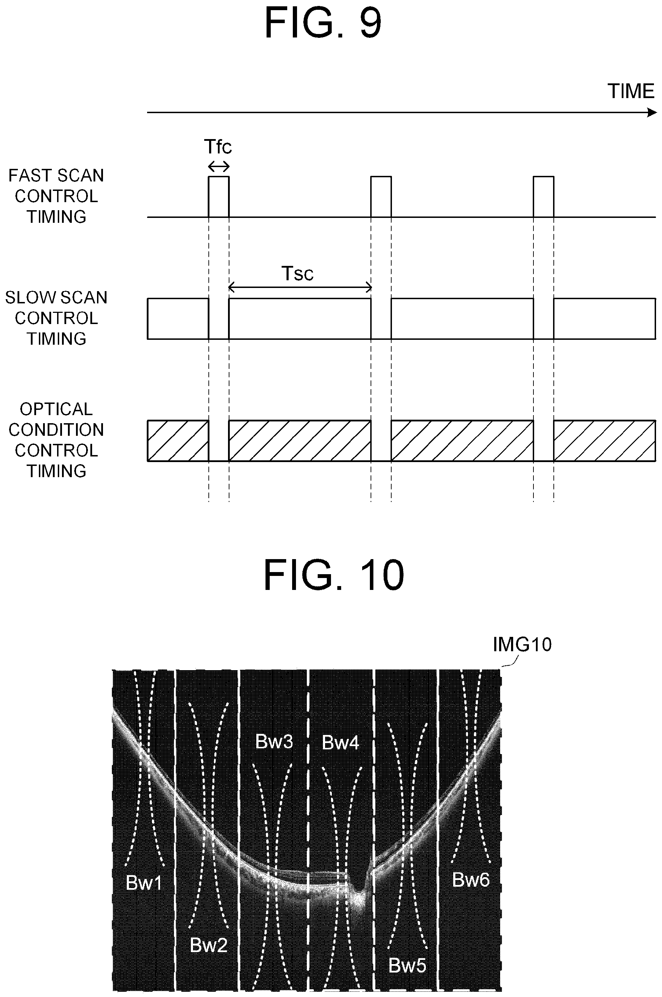

FIG. 9 represents a timing diagram showing an example of a change timing of the optical system during a single scan performed in the ophthalmic apparatus 1 according to the first embodiment. In FIG. 9, a horizontal axis represents time, and a vertical axis schematically represents a signal level of the control signal for controlling each control timing.

The main controller 211 changes the optical condition(s) of the optical system in synchronization with the control timing of the slow scan (period of slow scan) during a single scan performed by combining the fast scan (circle scan) controlled with the period Tfc and the slow scan (spiral scan) controlled with the period Tsc (> Tfc), as shown in FIG. 9. The change timing of the optical condition(s) of the optical system may be between timings of performing two consecutive A-scans.

In other words, the main controller 211 sequentially causes OCT scans shown in FIG. 6 to be performed on the scan region set in advance to the fundus Ef. The main controller 211 controls the image forming unit 220 to form the OCT image each time the OCT data is acquired through the OCT scan. Further, the main controller 211 controls the analyzer 232 to analyze the image formed by the image forming unit 220, and to calculate the position of the site of interest in the OCT image and/or the image quality evaluation value of the OCT image. Subsequently, the main controller 211 changes the optical condition(s) of the optical system based on the position of the site of interest in the OCT image and/or the image quality evaluation value of the OCT image in synchronization with a start timing for the slow scan in the next OCT scan.

FIG. 10 schematically shows a focusing position of the measurement light LS different for each scan region in the B-scan image acquired by the ophthalmic apparatus 1 according to the first embodiment. In FIG. 10, the focusing positions of the measurement light LS in the segmented image obtained by segmenting the B-scan image IMG10 corresponding to the scan regions are illustrated as beam waist Bw1 to Bw6.

The B-scan image IMG10 shown in FIG. 10 is a tomographic image when the OCT focusing lens 45 is moved for each scan region so that focusing position of the measurement light LS is positioned on the retinal pigment epithelium layer or its vicinity in the fundus Ef.

For example, the main controller 211 causes OCT scan (for example, circle scan) to be performed at a scan position different from the fundus Ef in the B-scan image IMG10 shown in FIG. 10, in advance. And, the main controller 211 moves the OCT focusing lens 45 in synchronization with the start timing for the slow scan for each scan region, based on this OCT data or the B-scan image formed based on the OCT data. Thereby, the tomographic image of the fundus Ef can be acquired with good image quality, even when the OCT scan is performed on the fundus Ef at a wide angle.

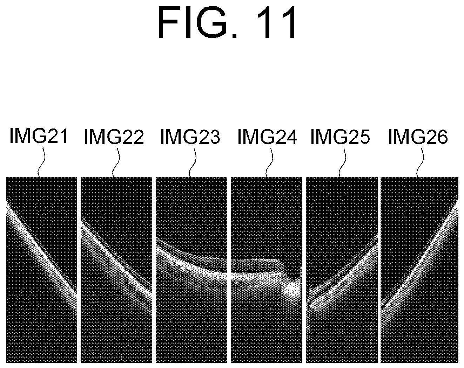

FIG. 11 schematically shows a tomographic image when the optical path length difference between the measurement light LS and the reference light LR is changed for each scan region by the ophthalmic apparatus 1 according to the first embodiment. In FIG. 11, the segmented images IMG21 to IMG 26 obtained by segmenting the B-scan image corresponding to the scan regions are illustrated.

For example, the main controller 211 causes OCT scan (for example, circle scan) to be performed in advance. And, the main controller 211 controls at least one of the optical path length changing unit 41, or, the corner cube 114 and reference driver 114A in synchronization with the start timing for the slow scan for each scan region, based on this OCT data or the B-scan image formed based on the OCT data. In this case, the main controller 211 controls the image synthesizing unit 231 to generate the synthetic image in which the site of interest (retinal pigment epithelium layer) in the fundus Ef is smoothly connected by performing position matching of the segmented images IMG21 to IMG 26 in the depth direction. Thereby, the tomographic image of the fundus Ef can be acquired with good image quality, even when the OCT scan is performed on the fundus Ef at a wide angle.

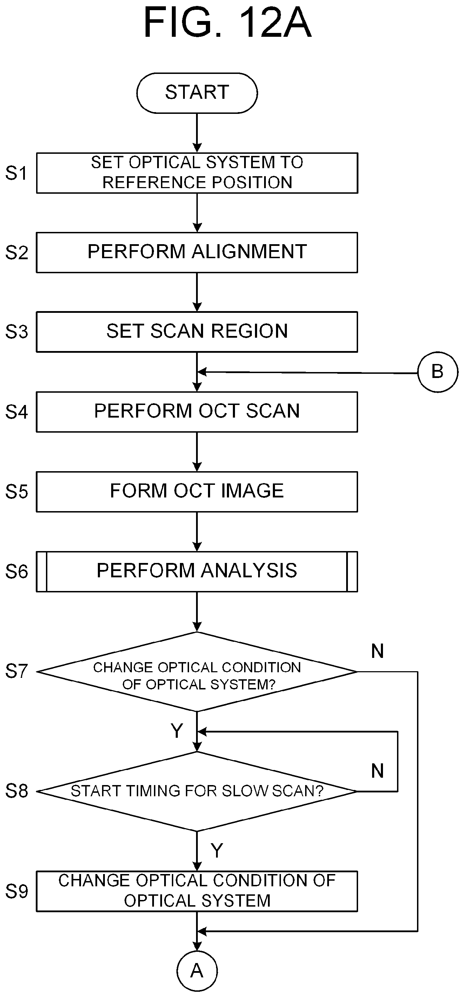

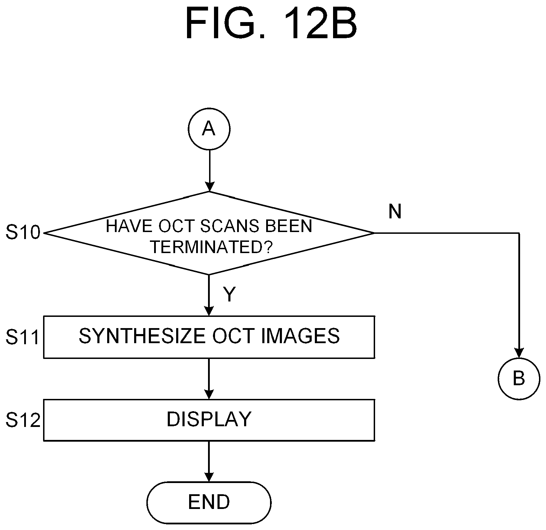

FIG. 12A and FIG. 12B show flowcharts of the example of the operation of the ophthalmic apparatus 1 according to the first embodiment. The storage unit 212 stores computer program(s) for realizing the processing shown in FIG. 12A and FIG. 12B. The main controller 211 operates according to the computer programs, and thereby the main controller 211 performs the processing shown in FIG. 12A and FIG. 12B.

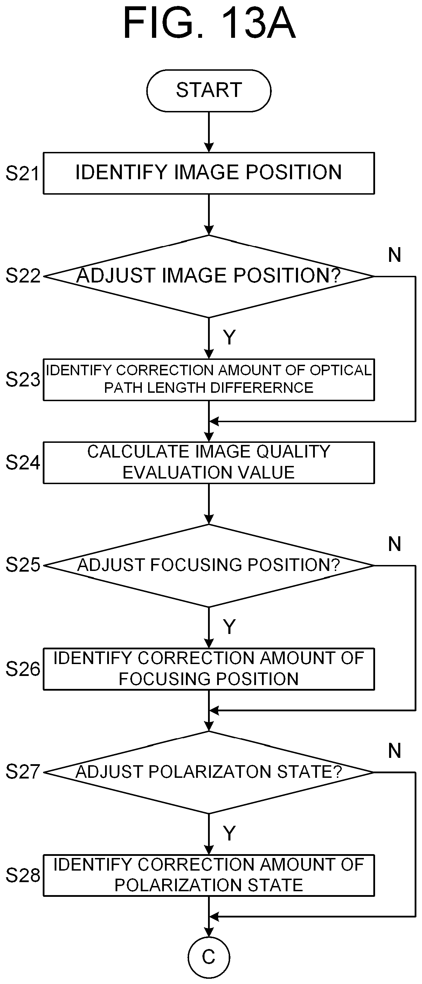

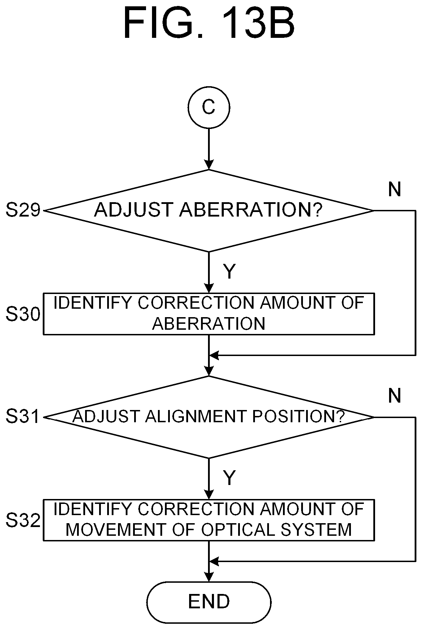

FIG. 13A and FIG. 13B show flowcharts of examples of the operation of step S6 in FIG. 12A. The storage unit 212 stores computer program(s) for realizing the processing shown in FIG. 13A and FIG. 13B. The main controller 211 operates according to the computer programs, and thereby the main controller 211 performs the processing shown in FIG. 13A and FIG. 13B.

### (S1: Set optical system to reference position)

As shown in FIG. 12A, first, the main controller 211 sets the optical conditions of the optical system constituting the ophthalmic apparatus 1 to a predetermined initial conditions, and sets the optical system to a reference position based on the set initial conditions.

### (S2: Perform alignment)

Subsequently, the main controller 211 performs alignment adjustment of the optical system relative to the eye E to be examined in a state where the fixation target is presented at a predetermined fixation position. Examples of the alignment adjustment include manual alignment and automatic alignment.

When the alignment adjustment is performed manually, the main controller 211 controls the alignment optical system 50 to project a pair of alignment indicators onto the eye E to be examined. A pair of alignment bright spots are displayed on the display unit 240A as the light receiving images of these alignment indicators. Further, the main controller 211 displays an alignment scale representing the target position of movement of the pair of alignment bright spots on the display unit 240A. The alignment scale is, for example, a bracket type image.

When the positional relationship between the eye E to be examined and the fundus camera unit 2 (objective lens 22) is appropriate, that is, when the distance (working distance) between the eye E to be examined and the fundus camera unit 2 is appropriate and the optical axis of the optical system of the fundus camera unit 2 and the ocular axis (corneal apex position) of the eye E to be examined are (approximately) coincident, the pair of alignment bright spots are formed at a predetermined position (for example, intermediate position between the corneal apex and the center of corneal curvature) respectively, and is projected onto the eye E to be examined, according to a known method. The examiner (user) can perform the alignment adjustment of the optical system to the eye E to be examined by moving the fundus camera unit 2 three-dimensionally so as to guide the pair of alignment bright spots into the alignment scale.

When the alignment adjustment is performed automatically, the movement mechanism 150 for moving the fundus camera unit 2 is used. The data processor 230 identifies the position of each alignment bright spot in the screen displayed on display unit 240A, and obtains a displacement between the identified position of each alignment bright point and the alignment scale. The main controller 211 controls the movement mechanism 150 to move the fundus camera unit 2 so as to cancel this displacement. Identifying the position of each alignment bright spot can be performed, for example, by obtaining the luminance distribution of each alignment bright spot and obtaining the position of the center of gravity based on this luminance distribution. Since the position of the alignment scale is constant, the desired displacement can be obtained, for example, by calculating the displacement between the center position of the alignment scale and the above position of the center of gravity. The movement direction and the movement distance of the fundus camera unit 2 can be determined by referring to a preset unit movement distances in the x-direction, y-direction, and z-direction (e.g., the result of prior measurement of how much the alignment indicator moves in which direction, when the fundus camera unit 2 is moved by how much in which direction). The main controller 211 generates signals according to the determined movement direction and movement distance, and transmits these signals to the movement mechanism 150. Thereby, the position of the optical system relative to the eye E to be examined is changed automatically.

### (S3: Set scan region)

The main controller 211 sets the scan region so that the site of interest in the eye E to be examined is positioned at a scan center position (position corresponding to the optical axis of the optical system). For example, the main controller 211 sets the ammonite scan as the scan mode, and sets a plurality of scan regions to be scanned, as shown in FIG. 6 or FIG. 8.

### (S4: Perform OCT scan)

Subsequently, the main controller 211 controls the optical scanner 42, the OCT unit 100, and the like to perform ammonite scan on the region set in step S3. It is assumed that, in step S4, the ammonite scan is performed across the plurality of scan regions as shown in FIG. 8.

### (S5: Form OCT image)

Next, the main controller 211 controls the image forming unit 220 to sequentially form the OCT images based on the OCT data acquired by performing OCT scans on the plurality of scan regions sequentially performed in step S4.

In some embodiments, the main controller 211 controls the image synthesizing unit 231 to perform position matching of the plurality of OCT images formed in step S5 to generate the synthetic image. The synthetic image is one of the OCT images.

### (S6: Perform analysis)

Subsequently, the main controller 211 controls the analyzer 232 to perform analysis processing on the OCT image (or synthetic image) formed in step S5. In step S6, the position of the site of interest in the OCT image or the synthetic image formed in step S5, or the image quality evaluation value of the OCT image or the synthetic image is calculated.

The details of step S6 will be described below.

### (S7: Change optical condition of optical system?)

Subsequently, the main controller 211 determined whether or not the optical condition(s) of the optical system should be changed based on the processing result obtained by performing analysis processing executed in step S6. For example, the main controller 211 determines whether or not the optical condition(s) of the optical system should be changed by determining whether or not there is optical condition of the optical system that should be changed as a result of the analysis processing in step S6.

Specifically, the main controller 211 determines that the optical condition should be changed when at least one of correction amount of the optical path length difference between the measurement light LS and the reference light LR, correction amount of the focusing position of the measurement light LS, correction amount of the polarization state of the light L0 from the light source unit 101 or the measurement light LS, aberration correction amount, or correction amount of movement of the optical system is identified. On the other hand, when none of the above correction amounts are identified, the main controller 211 determines that the optical condition(s) of the optical system should not be changed.

When it is determined in step S7 that the optical condition(s) of the optical system should be changed (S7: Y), the operation of the ophthalmic apparatus 1 proceeds to step S8. When it is determined in step S7 that the optical condition(s) of the optical system should not be changed (S7: N), the operation of the ophthalmic apparatus 1 proceeds to step S10.

### (S8: Start timing for slow scan?)

When it is determined in step S7 that the optical condition(s) of the optical system should be changed (S7: Y), the main controller 211 determines whether or not it is a start timing for the slow scan that constitutes the ammonite scan. For example, the main controller 211 determines whether or not it is the start timing for the slow scan (spiral scan) based on the control content to the optical scanner 42 for performing OCT scan on the scan region set in step S3.

When it is determined in step S8 that it is the start timing for the slow scan (S8: Y), the operation of the ophthalmic apparatus 1 proceeds to step S9. When it is determined in step S8 that it is not the start timing for the slow scan (S8: N), the operation of the ophthalmic apparatus 1 repeats step S8.

### (S9: Change optical condition of optical system)

When it is determined in step S8 that it is the start timing for the slow scan (S8: Y), the main controller 211 changes the optical condition(s) of the optical system.

For example, the main controller 211 controls the OCT focusing driver 45A to change the optical condition(s) of the optical system by moving the position of the OCT focusing lens 45 on the optical axis by a movement amount corresponding to the correction amount of the focusing position identified in step S6.

For example, the main controller 211 controls the polarization controller 103 to change the optical condition(s) of the optical system by changing the polarization component of the light L0 by a change amount corresponding to the correction amount of the polarization state identified in step S6.

For example, the main controller 211 controls the VCC driver 47A to change the optical condition(s) of the optical system by changing the cylindrical power of the VCC lens 47 or the cylindrical axis angle of the VCC lens 47 by the change amount corresponding to the aberration correction amount identified in step S6.

For example, the main controller 211 controls the optical path length changing unit 41 or the reference driver 114A to change the optical condition(s) of the optical system by changing the position of the optical path length changing unit 41 (corner cube) on the optical path of the measurement light LS or the position of the corner cube 114 on the optical path of the reference light LR by the correction amount of the optical path length difference identified in step S6.

For example, the main controller 211 controls the movement mechanism 150 to change the optical condition(s) of the optical system by changing the position in the optical axis direction of the optical system relative to the eye E to be examined or the position in the direction intersecting the optical axis direction of the optical system by the movement correction amount of the optical system identified in step S6.

### (S10: Have OCT scans been terminated?)

Subsequent to step S9, the main controller 211 determines whether or not to terminate the OCT scans. For example, the main controller 211 determines whether or not to terminate the OCT scans based on the control content to the optical scanner 42 for performing OCT scans on the scan region set in step S3.

When it is determined in step S10 that the OCT scans are terminated (S10: Y), the operation of the ophthalmic apparatus 1 proceeds to step S11. When it is determined in step S10 that the OCT scans are not terminated (S10: N), the operation of the ophthalmic apparatus 1 proceeds to step S4.

### (S11: Synthesize OCT images)

When it is determined in step S10 that the OCT scans are terminated (S10: Y), the main controller 211 controls the image synthesizing unit 231 to perform position matching of the plurality of OCT images formed in step S5 to generate the synthetic image. When the synthetic image has already been generated, step S11 may be omitted.

### (S12: Display)

Subsequent to step S11, the main controller 211 causes the synthetic image generated in step S11 to be displayed on the display unit 240A (display apparatus 3), as a display controller.

In some embodiments, the main controller 211 causes the correction amount (adjustment amount) of the optical condition of the optical system identified in step S6 to be displayed on the display unit 240A for each scan region.

This terminates the operation of the ophthalmic apparatus 1 (END).

In step S6 in FIG. 12A, the processing shown in FIG. 13A and FIG. 13B is performed on the OCT image formed in step S5. It is assumed that, prior to the processing in step S6, the image synthesizing unit 231 generates the synthetic image from the plurality of OCT images formed in step S5.

### (S21: Identify image position)

In step S6 of FIG. 12A, the main controller 211 controls the image position identifying unit 232A to identify the position in the depth direction (rendering position) of the site of interest in the OCT image formed in step S5.

For example, the image position identifying unit 232A identifies a position of the center of gravity in the depth direction in the OCT image, and identifies the position in the depth direction of the site of interest in the OCT image based on the identified position of the center of gravity, as described above.

### (S22: Adjust image position?)

Next, the main controller 211 determines whether or not the rendering position in the depth direction of the site of interest in the OCT image identified in step S21 should be adjusted. For example, the main controller 211 determines whether or not the rendering position in the depth direction of the site of interest in the OCT image identified in step S21 should be adjusted, by determining whether or not the rendering position in the depth direction of the site of interest in the OCT image identified in step S21 is within a predetermined depth range.

When it is determined in step S22 that the rendering position in the depth direction of the site of interest in the OCT image should be adjusted (S22: Y), the processing in step S6 proceeds to step S23. When it is determined in step S22 that the rendering position in the depth direction of the site of interest in the OCT image should not be adjusted (S22: N), the processing in step S6 proceeds to step S24.

### (S23: Identify correction amount of optical path length difference)

When it is determined in step S22 that the rendering position in the depth direction of the site of interest in the OCT image should be adjusted (S22: Y), the main controller 211 identifies a correction amount of the optical path length difference between the measurement light LS and the reference light LR.

For example, the main controller 211 identifies the correction amount of the optical path length difference between the measurement light LS and the reference light LR, based on a displacement of the rendering position in the depth direction of the site of interest in the OCT image identified in step S21 relative to a reference depth position within a predetermined depth range. In other words, the main controller 211 identifies the correction amount of the optical path length difference between the measurement light LS and the reference light LR so that the rendering position in the depth direction of the site of interest in the OCT image identified in step S21 coincides with the reference depth position within the predetermined depth range. Examples of the reference depth position include a center position (or position of the center of gravity) within a predetermined depth range, an upper limit position within a predetermined reference range within the depth range described above, and a lower limit position within a predetermined reference range within the depth range described above.

### (S24: Calculate image quality evaluation value)

When it is determined in step S22 that the rendering position in the depth direction of the site of interest in the OCT image should not be adjusted (S22: N), or subsequent to step S23, the main controller 211 controls the image quality evaluation value calculator 232B to calculate the image quality evaluation value of the OCT image (synthetic image) formed in step S5.

For example, the main controller 211 controls the image quality evaluation value calculator 232B to calculate the image quality evaluation value, for each of the plurality of OCT images formed for each scan region in step S5.

### (S25: Adjust focusing position?)

Subsequent to step S24, the main controller 211 determines whether or not the focusing position of the measurement light LS should be adjusted based on the image quality evaluation value calculated in step S24. In the present embodiment, the main controller 211 determines whether or not the focusing position of the measurement light LS for each of the plurality of OCT images based on the image quality evaluation value calculated for each of the plurality of OCT images in step S24.

For example, the main controller 211 determines whether or not the focusing position of the measurement light LS should be adjusted based on a comparison result between a predetermined reference image quality evaluation value and the image quality evaluation value calculated in step S24.

When it is determined in step S25 that the focusing position of the measurement light LS should be adjusted (S25: Y), the processing in step S6 proceeds to step S26. When it is determined in step S25 that the focusing position of the measurement light LS should not be adjusted (S25: N), the processing in step S6 proceeds to step S27.

### (S26: Identify correction amount of focusing position)

When it is determined in step S25 that the focusing position of the measurement light LS should be adjusted (S25: Y), the main controller 211 identifies a correction amount of the focusing position of the measurement light LS.

For example, the main controller 211 compares a predetermined reference image quality evaluation value with the image quality evaluation value calculated in step S24, and identifies the correction amount of the focusing position of the measurement light LS for each of the plurality of OCT images so that the image quality of the OCT image formed in step S5 is higher than the image quality corresponding to the reference image quality evaluation value. In some embodiments, the main controller 211 identifies an adjustment direction of the focusing position of the measurement light LS, based on the displacement of the scan region relative to the deepest portion of the cross-sectional shape of the fundus Ef, and identifies the correction amount of the focusing position of the measurement light LS in the identified adjustment direction.

### (S27: Adjust polarization state?)

When it is determined in step S25 that the focusing position of the measurement light LS should not be adjusted (S25: N), or subsequent to step S26, the main controller 211 determines whether or not the polarization state of the light L0 from the light source unit 101 or the measurement light LS should be adjusted based on the image quality evaluation value calculated in step S24. In the present embodiment, the main controller 211 determines whether or not the polarization state of the light L0 or the measurement light LS should be adjusted for each of the plurality of OCT images, based on the image quality evaluation value calculated for each of the plurality of OCT images in step S24.

For example, the main controller 211 determines whether or not the polarization state of the light L0 or the measurement light LS should be adjusted based on a comparison result between a predetermined reference image quality evaluation value and the image quality evaluation value calculated in step S24. In some embodiments, polarization information, in which the comparison results between the reference image quality evaluation values and the calculated image quality evaluation values are associated with the polarization states, is stored in advance in the storage unit 212, and the main controller 212 determines whether or not the polarization state of the light L0 or the measurement light LS should be adjusted by referring to the polarization information stored in the storage unit 212.

When it is determined in step S27 that the polarization state of the light L0 or the measurement light LS should be adjusted (S27: Y), the processing in step S6 proceeds to step S28. When it is determined in step S27 that the polarization state of the light L0 or the measurement light LS should not be adjusted (S27: N), the processing in step S6 proceeds to step S29.

### (S28: Identify correction amount of polarization state)

When it is determined in step S27 that the polarization state of the light L0 or the measurement light LS should be adjusted (S27: Y), the main controller 211 identifies the correction amount of the polarization state of the light L0 or the measurement light LS.

For example, the main controller 211 compares a predetermined reference image quality evaluation value with the image quality evaluation value calculated in step S24, and identifies the correction amount of the polarization state of the light L0 or the measurement light LS for each of the plurality of OCT images so that the image quality of the OCT image formed in step S5 is higher than the image quality corresponding to the reference image quality evaluation value. In some embodiments, the main controller 211 identifies an adjustment direction of the polarization state of the measurement light LS of the light L0, and identifies the correction amount of the polarization state of the light L0 or the measurement light LS in the identified adjustment direction, by referring to the polarization information described above.

### (S29: Adjust aberration?)

Subsequently, when it is determined in step S27 that the polarization state of the light L0 or the measurement light LS should not be adjusted (S27: N), or subsequent to step S28, the main controller 211 determines whether or not the aberration should be adjusted based on the image quality evaluation value calculated in step S24. In the present embodiment, the main controller 211 determines whether or not the aberration should be adjusted for each of the plurality of OCT images based on the image quality evaluation value calculated for each of the plurality of OCT images in step S24.

For example, the main controller 211 determines whether or not the aberration should be adjusted based on a comparison result between a predetermined reference image quality evaluation value and the image quality evaluation value calculated in step S24. In some embodiments, aberration information, in which the comparison results between the reference image quality evaluation values and the calculated image quality evaluation values are associated with the aberrations, is stored in advance in the storage unit 212, and the main controller 212 determines whether or not the aberration should be adjusted by referring to the aberration information stored in the storage unit 212.

When it is determined in step S29 that the polarization state of the light L0 or the measurement light LS should be adjusted (S29: Y), the processing in step S6 proceeds to step S30. When it is determined in step S29 that the aberration should not be adjusted (S29: N), the processing in step S6 proceeds to step S31.

### (S30: Identify correction amount of aberration)

When it is determined in step S29 that the aberration should be adjusted (S29: Y), the main controller 211 identifies a correction amount of the aberration.

For example, the main controller 211 compares a predetermined reference image quality evaluation value with the image quality evaluation value calculated in step S24, and identifies the correction amount of the aberration for each of the plurality of OCT images so that the image quality of the OCT image formed in step S5 is higher than the image quality corresponding to the reference image quality evaluation value. In some embodiments, the main controller 211 identifies an adjustment direction of the polarization state of the measurement light LS of the light L0, and identifies the correction amount of the aberration in the identified adjustment direction, by referring to the aberration information described above.

### (S31: Adjust alignment position?)

Subsequent to step S30, when it is determined in step S29 that the aberration should not be adjusted (S29: N), or subsequent to step S30, the main controller 211 determines whether or not the alignment position of the optical system relative to the eye E to be examined should be adjusted based on the image quality evaluation value calculated in step S24.

In some embodiments, the main controller 211 determines whether or not the alignment position should be adjusted for each of the plurality of OCT images based on the image quality evaluation value calculated for each of the plurality of OCT images in step S24. For example, the main controller 211 determines whether or not the alignment position should be adjusted based on a comparison result between a predetermined reference image quality evaluation value and the image quality evaluation value calculated in step S24.

In some embodiments, the main controller 211 determines whether or not the alignment position should be adjusted based on the rendering position of the site of interest in the OCT image identified by the image position identifying unit 232A. For example, the main controller 211 determines whether or not the alignment position should be adjusted based on the displacement of the position of the site of interest in the OCT image identified by the image position identifying unit 232A relative to a predetermined reference position.

When it is determined in step S31 that the alignment position should be adjusted (S31: Y), the processing in step S6 proceeds to step S32. When it is determined in step S31 that the alignment position should not be adjusted (S31: N), the processing in step S6 is terminated (END).

### (S32: Identify correction amount of movement of optical system)

When it is determined in step S31 that the alignment position should be adjusted (S32:Y), the main controller 211 identifies a correction amount of the movement of the optical system.

For example, the main controller 211 compares a predetermined reference image quality evaluation value with the image quality evaluation value calculated in step S24, and identifies the correction amount of the movement of the optical system so that the image quality of the OCT image formed in step S5 is higher than the image quality corresponding to the reference image quality evaluation value. In some embodiments, the main controller 211 identifies the correction amount of the movement of the optical system for each scan region. In some embodiments, the main controller 211 identifies a single correction amount of the movement of the optical system so that the statistical value (for example, average value) of the image quality evaluation values becomes maximum.

For example, the main controller 211 identifies the correction amount of the movement of the optical system based on the displacement of the position of the site of interest in the OCT image identified by the image position identifying unit 232A relative to a predetermined reference position.

When it is determined in step S31 that the alignment position should not be adjusted (S31: N), or subsequent to step S32, the processing in step S6 is terminated (END).

It should be noted that the order of change of the optical conditions of the optical system according to the embodiments is not limited to the order of change shown in FIG. 13A and FIG. 13B. Further, the optical condition(s) of the optical system changed in the embodiments may include any one or more of the optical conditions shown in FIG. 13A and FIG. 13B.

As described above, according to the first embodiment, in case of performing OCT scan on the eye E to be examined by combining the slow scan and the fast scan, the cross-sectional structure of the eye E to be examined is substantially estimated based on the OCT data or the OCT image, which is acquired by the fast scan that has been performed in past, and the optical condition(s) of the optical system is/are changed in synchronization with the period of the slow scan based on the estimated cross-sectional structure. As a result, the images of the eye E to be examined can be acquired with good image quality in case of imaging the eye E to be examined at a wide angle, without performing pre-scan.

### <Second embodiment>

In the first embodiment, a case where the VCC lens 47 is provided in the ophthalmic apparatus as the aberration correction device has been described. However, the configuration according to the embodiments is not limited to this. For example, the ophthalmic apparatus according to the embodiments may be configured to include a wavefront aberration correction optical system as the aberration correction device.

Hereinafter, the configuration according to the second embodiment will be described, focusing on the differences from the configuration according to the first embodiment.

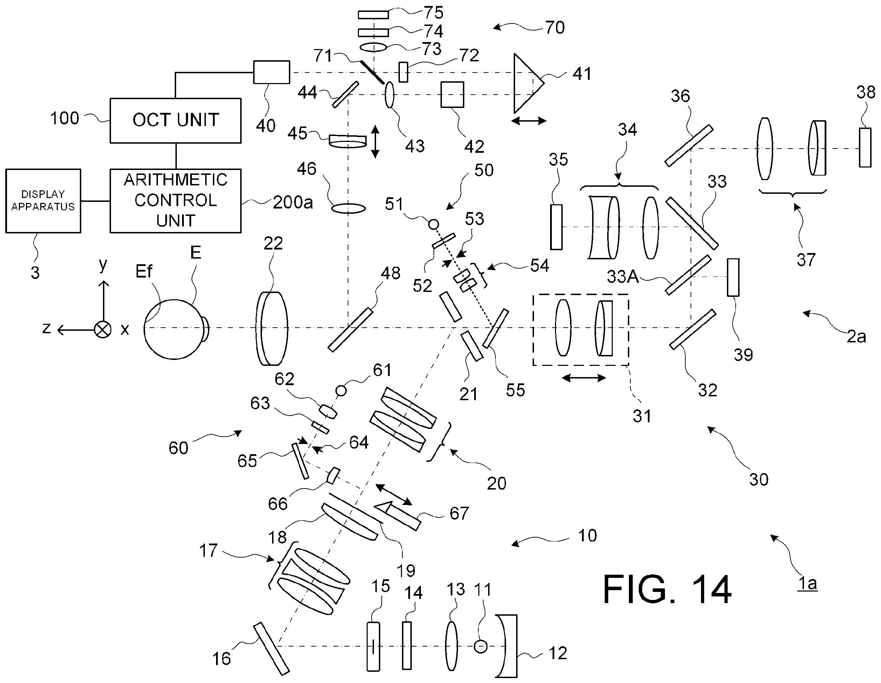

FIG. 14 shows an example of a configuration of an optical system of an ophthalmic apparatus 1a according to the second embodiment. In FIG. 14, the same parts as in FIG. 1 are shown by the same symbols and their descriptions will be omitted in an appropriate manner.

The configuration of the optical system in the ophthalmic apparatus 1a according to the second embodiment differs from that in the ophthalmic apparatus 1 according to the first embodiment in that the fundus camera unit 2a is provided instead of the fundus camera unit 2 and the arithmetic control unit 200a is provided instead of the arithmetic control unit 200.

The configuration of the fundus camera unit 2a differs from that of the fundus camera unit 2 in that a wavefront aberration correction optical system 70 is provided instead of the VCC lens 47. The configuration of the arithmetic control unit 200a differs from that of the arithmetic control unit 200 in that the arithmetic control unit 200a controls the wavefront aberration correction optical system 70 instead of the VCC lens 47.

The wavefront aberration correction optical system 70 includes a beam splitter 71, a transmissive adaptive optical element 72, a relay lens 73, a lens array 74, and a wavefront sensor 75.

In FIG. 14, the beam splitter 71 and the adaptive optical element 72 are arranged between the collimator lens unit 40 and the optical path length changing unit 41. The adaptive optical element 72 is arranged between the beam splitter 71 and the optical path length changing unit 41.

The beam splitter 71 splits the returning light of the measurement light LS into light guided to the collimator lens unit 40 side and light guided to the relay lens 73 side. The beam splitter 71 may be capable of changing a split ratio (branching ratio) of these lights. For example, when controlling the adaptive optical element 72 described below (for example, when performing the provisional measurement), the returning light of the measurement light LS is split into two lights with the split ratio of "50:50". And, when not controlling the adaptive optical element 72 (for example, when performing the main measurement), the returning light of the measurement light LS can be split into two lights with the split ratio of "90:10" so that the light quantity of the light guided to the collimator lens unit 40 side is increased.

The adaptive optical element 72 may be deformable phase plate (DPP), for example. The adaptive optical element 72 is an optical element that can locally change the phase of the wavefront of light passing through it.

In a reflection direction of the beam splitter 71, the relay lens 73 , the lens array 74, and the wavefront sensor 75 are arranged. Each of the adaptive optical element 72 and the lens array 74 is arranged at a position substantially conjugate optically to a pupil of the eye E to be examined (pupil conjugate position) or in the vicinity thereof.

The beam splitter 71 transmits the measurement light LS from the collimator lens unit 40 to guide the measurement light LS to the adaptive optical element 72, and guides the returning light of the measurement light LS from the eye E to be examined that has passed through the adaptive optical element 72 to the relay lens 73. The adaptive optical element 72 corrects at least the wavefront aberration of the returning light of the measurement light LS. The returning light of the measurement light LS that has been guided to the relay lens 73 passes through the relay lens 73, and is guided to the lens array 74.

The lens array 74 includes a plurality of lenses arranged in an array form, and generates a plurality of converging rays from the light passing through the relay lens 73. The plurality of converging rays generated by the lens array 74 is irradiated onto a sensor surface of the wavefront sensor 75. The wavefront sensor 75 may be a CMOS (complementary metal-oxide semiconductor) image sensor or a CCD (charge-coupled device) image sensor.

The wavefront of the light passing through the adaptive optical element 72 can be locally changed in accordance with detection results of the plurality of converging rays obtained by the wavefront sensor 75. Thereby, the adaptive optical element 72 can correct the wavefront aberration of the returning light of the measurement light LS from the eye E to be examined.

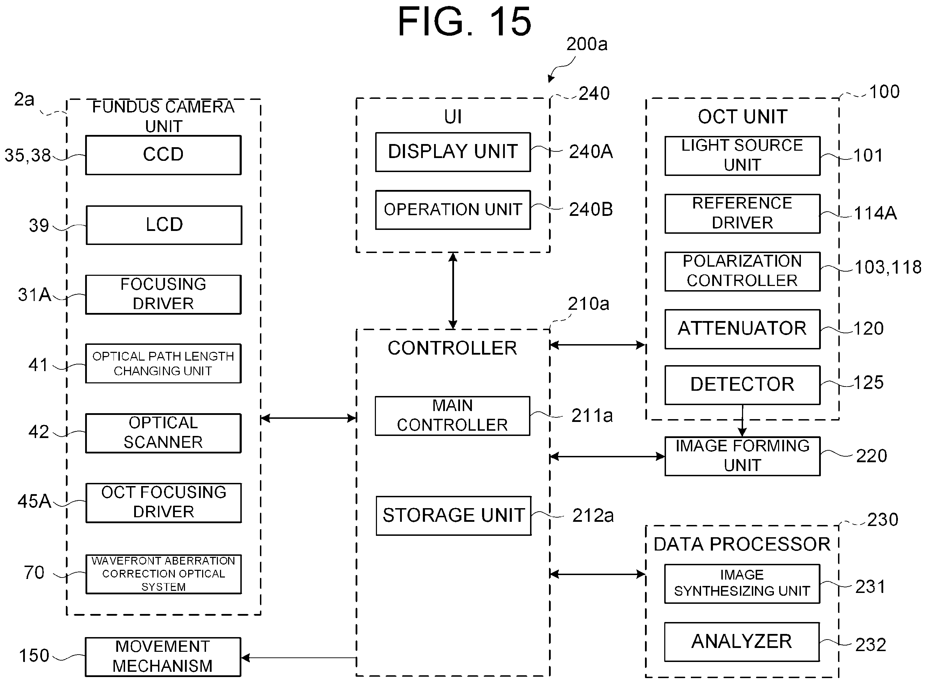

FIG. 15 shows a block diagram of an example of a configuration of a processing system of the ophthalmic apparatus 1a according to the second embodiment. In FIG. 15, the same parts as in FIG. 4 are shown by the same symbols and their descriptions will be omitted in an appropriate manner.

The configuration of the processing system of the ophthalmic apparatus 1a according to the second embodiment differs from the configuration of the processing system of the ophthalmic apparatus 1 according to the first embodiment mainly in that the wavefront aberration correction optical system 70 is provided instead of the VCC lens 47 and a controller 210a is provided instead of the controller 210.

The controller 210a includes a main controller 211a and a storage unit 212a. The controller 210a differs from the controller 210 in that the controller 210a controls the wavefront aberration correction optical system 70 instead of the VCC driver 47A.

In other words, according to the second embodiment, by controlling the wavefront aberration correction optical system 70 to change the aberration correction component, the optical condition(s) of the optical system can be changed.

The wavefront aberration correction optical system 70 is an example of the "aberration correction device" according to the embodiments. The optical system included in the OCT unit 100, the optical path length changing unit 41, the OCT focusing lens 45, and the wavefront aberration correction optical system 70 (interference optical system) are an example of the "optical system" according to the embodiments.

### <Modification example>

In the first embodiment or the second embodiment, the ammonite scan is described as an example of a scan that combines the slow scan and the fast scan. However, the embodiments are not limited to this.

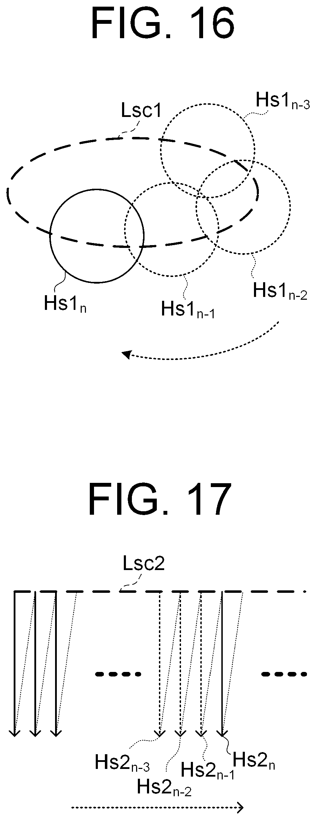

FIG. 16 shows an explanatory diagram of an XY scan according to a first modification example of the first embodiment or the second embodiment.

The XY scan according to the first modification example is also a scan mode in which, while the scan center position of the fast scan is moved along the scan pattern (scan trajectory) of the slow scan, the one or more fast scans are sequentially performed with a scan pattern defined with reference to the moved scan center position. In the XY scan according to the present modification example, the fast scan is a circle scan, the slow scan is an ellipse scan, and the circle scans, which are sequentially performed, are performed so that the circle scans intersect the scan pattern of the previous circle scan. In other words, the circle scan are sequentially performed so that the scan patterns of two adjacent circle scans intersect.

In FIG. 16, a scan pattern Lsc1 of the slow scan is schematically represented and fast scan patterns HS1ₙ (n is an integer of 4 or more), HS1ₙ₋₁, HS1ₙ₋₂, and HS1ₙ₋₃ are schematically represented.

The XY scan, similar to the ammonite scan, can perform scan on the eye E to be examined from a variety of scan directions with a simple control, by combining the slow scan and the fast scan. In particular, the scanning can be performed around near the center of the XY scan (scan center position of slow scan) at high density, and a wide-angle region including the site of interest can be scanned with simple control.

According to the present modification example, the eye E to be examined can be measured or imaged at a higher quality and wide angle regardless of the cross-sectional structure of the eye E to be examined, by scanning the eye E to be examined with the XY scan.

FIG. 17 shows an explanatory diagram of a three-dimensional vertical (3D (V)) scan according to a second modification example of the first embodiment or the second embodiment.

The 3D (V) scan according to the second modification example is also a scan mode in which, while the scan center position of the fast scan is moved along the scan pattern (scan trajectory) of the slow scan, the one or more fast scans are sequentially performed with a scan pattern defined with reference to the moved scan center position. In the 3D (V) scan according to the present modification example, the fast scan is a line scan in the vertical direction, and the slow scan is a line scan in the horizontal direction.

In FIG. 17, a scan pattern Lsc2 of the slow scan is schematically represented and fast scan patterns HS2ₙ (n is an integer of 4 or more), HS2ₙ₋₁, HS2ₙ₋₂, and HS2ₙ₋₃ are schematically represented.

The 3D (V) scan, similar to the ammonite scan, can perform scan on the eye E to be examined from a variety of scan directions with a simple control, by combining the slow scan and the fast scan. In particular, the scanning can be performed at a uniform density on the eye E to be examined, and a wide-angle region including the site of interest can be scanned with simple control.

According to the present modification example, the eye E to be examined can be measured or imaged at a higher quality and wide angle regardless of the cross-sectional structure of the eye E to be examined, by scanning the eye E to be examined with the 3D (V) scan.

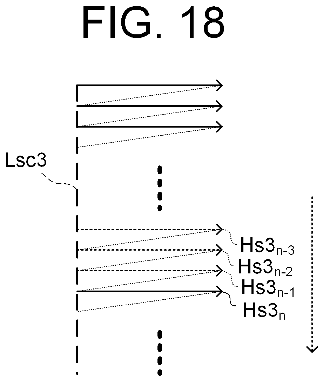

FIG. 18 shows an explanatory diagram of a three-dimensional horizontal (3D (H)) scan according to a third modification example of the first embodiment or the second embodiment.

The 3D (H) scan according to the third modification example is also a scan mode in which, while the scan center position of the fast scan is moved along the scan pattern (scan trajectory) of the slow scan, the one or more fast scans are sequentially performed with a scan pattern defined with reference to the moved scan center position. In the 3D (H) scan according to the present modification example, the fast scan is a line scan in the horizontal direction, and the slow scan is a line scan in the vertical direction.

In FIG. 18, a scan pattern Lsc3 of the slow scan is schematically represented and fast scan patterns HS3ₙ (n is an integer of 4 or more), HS3ₙ₋₁, HS3ₙ₋₂, and HS3ₙ₋₃ are schematically represented.

The 3D (H) scan, similar to the ammonite scan, can perform scan on the eye E to be examined from a variety of scan directions with a simple control, by combining the slow scan and the fast scan. In particular, the scanning can be performed at a uniform density on the eye E to be examined, and a wide-angle region including the site of interest can be scanned with simple control.

According to the present modification example, the eye E to be examined can be measured or imaged at a higher quality and wide angle regardless of the cross-sectional structure of the eye E to be examined, by scanning the eye E to be examined with the 3D (H) scan.

### [Actions]

The OCT apparatus, the method of controlling the OCT apparatus, and the program according to the embodiments will be explained.

The first aspect of the embodiments is an OCT apparatus (ophthalmic apparatus 1, 1a) including an optical system (optical system included in the OCT unit 100, optical path length changing unit 41, OCT focusing lens 45, and VCC lens 47 (or wavefront aberration correction optical system 70), interference optical system), and a controller (210, 210a, main controller 211, main controller 211a). The optical system is configured to allow an optical condition (focusing position of measurement light LS, polarization component of measurement light LS, aberration correction component, optical path length difference between measurement light LS and reference light LR, or position of optical system relative to the eye E to be examined) to be changed, includes an optical scanner (42), and is configured to acquire OCT data of an object to be measured (eye E to be examined) by scanning the object to be measured with measurement light (LS) using the optical scanner. The controller is configured to control the optical scanner so as to scan the object to be measured with the measurement light along a fast scan axis with reference to a reference position moved along a slow scan axis. The controller is configured to change the optical condition in synchronization with a period of the slow scan along the slow scan axis.

According to such an aspect, the optical condition(s) of the optical system can be changed in synchronization with the period of the slow scan using the OCT data acquired during scanning. Thereby, the image of the object to be measured can be acquired with good image quality without degrading the image quality due to misalignment of the scan position during scanning, etc. In particular, in case of imaging the object to be measured at a wide angle, the image of the object to be measured can be acquired with good image quality without performing pre-scan.

In the second aspect of the embodiments, in the first embodiment, the optical system includes an interference optical system (optical system included in the OCT unit 100, optical path length changing unit 41, OCT focusing lens 45, and VCC lens 47 (or wavefront aberration correction optical system 70)), and an optical path length difference changing member (optical path length changing unit 41, or, corner cube 114 and reference driver 114A). The interference optical system is configured to split light (L0) from a light source (light source unit 101) into the measurement light and reference light (LR), to project the measurement light having traveled through a measurement optical path, on which one or more optical elements (OCT focusing lens 45, corner cube 114, optical path length changing unit 41, polarization controller 103) are arranged, onto the object to be measured, and to detect interference light (LC) between returning light from the object to be measured and the reference light having traveled through a reference optical path. The optical path length difference changing member is configured to change a difference between an optical path length of the measurement light and an optical path length of the reference light. The controller is configured to change the optical condition of the optical system by controlling at least one of the one or more optical elements or the optical path length difference changing member.

According to such an aspect, by controlling at least one of the one or more optical elements or optical path length difference changing member, the optical condition of the optical system is changed to perform OCT on the object to be measured. Thereby, the image of the object to be measured can be acquired with good image quality regardless of the cross-sectional shape of the object to be measured.

In the third aspect of the embodiments, in the second aspect, the one or more optical elements includes a focusing lens (OCT focusing lens 45) or an aberration correction device (VCC lens 47 or wavefront aberration correction optical system 70).

According to such an aspect, by controlling at least one of the one or more optical elements including the focusing lens or aberration correction device, or optical path length difference changing member, the optical condition of the optical system is changed to perform OCT on the object to be measured without performing pre-scan. Thereby, the image of the object to be measured can be acquired with good image quality regardless of the cross-sectional shape of the object to be measured, and the measurement time (imaging time) can be shortened.

The fourth aspect of the embodiments, in the second aspect or the third aspect, further includes an image forming unit (220), and an image quality evaluation value calculator (232B). The image forming unit is configured to form an image of the object to be measured based on the OCT data. The image quality evaluation value calculator is configured to calculate an evaluation value of image quality of the image of the object to be measured. The controller is configured to control the one or more optical elements based on the evaluation value.

According to such an aspect, the optical condition of the optical system is changed based on the evaluation value of the image quality of the image of the object to be measured. Thereby, the image of the object to be measured can be acquired with good image quality with simple control and high precision, regardless of the cross-sectional shape of the object to be measured.

The fifth aspect of the embodiments, in any one of the first aspect to the third aspect, further includes a movement mechanism (150) configured to relatively move the optical system relative to the object to be measured. The controller is configured to change the optical condition of the optical system by controlling the movement mechanism.

According to such an aspect, by relatively moving the optical system relative to the object to be measured, the optical condition of the optical system is changed to perform OCT on the object to be measured. Thereby, the image of the object to be measured can be acquired with good image quality regardless of the cross-sectional shape of the object to be measured, and the measurement time (imaging time) can be shortened.

The sixth aspect of the embodiments, in the second aspect or the third aspect, further includes an image forming unit (220). The image forming unit is configured to form an image of the object to be measured based on the OCT data. The controller is configured to control the optical path length difference changing member based a position of the object to be measured in the image.

According to such an aspect, the optical path length difference between the measurement light and the reference light is changed in synchronization with the period of the slow scan using the OCT data acquired during scanning. Thereby, the image of the object to be measured can be acquired with good image quality.

In the seventh aspect of the embodiments, in any one of the first aspect to the third aspect, the fast scan axis is a circular scan axis, and the slow scan axis is a spiral scan axis.

According to such an aspect, the region near the center (scan center position of slow scan) can be scanned at high density in a short period of time, and the farther away the scan position is from the center, the lower the density of the scan becomes in a longer period of time, which allows the scan of a wide region including the site of interest with a simple control.

The eighth aspect of the embodiments is a method of controlling an OCT apparatus (ophthalmic apparatus 1, 1a) including an optical system (optical system included in the OCT unit 100, optical path length changing unit 41, OCT focusing lens 45, and VCC lens 47 (or wavefront aberration correction optical system 70), interference optical system) configured to allow an optical condition to be changed, including an optical scanner (42), and configured to acquire OCT data of an object to be measured (eye E to be examined) by scanning the object to be measured with measurement light (LS) using the optical scanner. The method of controlling the OCT apparatus includes a control step and an optical condition changing step. The control step is performed to control the optical scanner so as to scan the object to be measured with the measurement light along a fast scan axis with reference to a reference position moved along a slow scan axis. The optical condition changing step is performed to change the optical condition in synchronization with a period of the slow scan along the slow scan axis.

According to such an aspect, the optical condition(s) of the optical system can be changed in synchronization with the period of the slow scan using the OCT data acquired during scanning. Thereby, the image of the object to be measured can be acquired with good image quality without degrading the image quality due to misalignment of the scan position during scanning, etc. In particular, in case of imaging the object to be measured at a wide angle, the image of the object to be measured can be acquired with good image quality without performing pre-scan.

In the ninth aspect of the embodiments, in the eighth aspect, the optical system includes an interference optical system (optical system included in the OCT unit 100, optical path length changing unit 41, OCT focusing lens 45, and VCC lens 47 (or wavefront aberration correction optical system 70)), and an optical path length difference changing member (optical path length changing unit 41, or, corner cube 114 and reference driver 114A). The interference optical system is configured to split light (L0) from a light source (light source unit 101) into the measurement light and reference light (LR), to project the measurement light having traveled through a measurement optical path, on which one or more optical elements (OCT focusing lens 45, corner cube 114, optical path length changing unit 41, polarization controller 103) are arranged, onto the object to be measured, and to detect interference light (LC) between returning light from the object to be measured and the reference light having traveled through a reference optical path. The optical path length difference changing member is configured to change a difference between an optical path length of the measurement light and an optical path length of the reference light. The optical condition changing step is performed to change the optical condition of the optical system by controlling at least one of the one or more optical elements or the optical path length difference changing member.

According to such an aspect, by controlling at least one of the one or more optical elements or optical path length difference changing member, the optical condition of the optical system is changed to perform OCT on the object to be measured. Thereby, the image of the object to be measured can be acquired with good image quality regardless of the cross-sectional shape of the object to be measured.

In the tenth aspect of the embodiments, in the ninth aspect, the one or more optical elements includes a focusing lens (OCT focusing lens 45) or an aberration correction device (VCC lens 47 or wavefront aberration correction optical system 70).

According to such an aspect, by controlling at least one of the one or more optical elements including the focusing lens or aberration correction device, or optical path length difference changing member, the optical condition of the optical system is changed to perform OCT on the object to be measured without performing pre-scan. Thereby, the image of the object to be measured can be acquired with good image quality regardless of the cross-sectional shape of the object to be measured, and the measurement time (imaging time) can be shortened.

The eleventh aspect of the embodiments, in the ninth aspect or the tenth aspect, further includes an image forming step and an image quality evaluation value calculation step. The image forming step is performed to form an image of the object to be measured based on the OCT data. The image quality evaluation value calculation step is performed to calculate evaluation value of image quality of the image of the object to be measured. The optical condition changing step is performed to control the one or more optical elements based on the evaluation value.

According to such an aspect, the optical condition of the optical system is changed based on the evaluation value of the image quality of the image of the object to be measured. Thereby, the image of the object to be measured can be acquired with good image quality with simple control and high precision, regardless of the cross-sectional shape of the object to be measured.

In the twelfth aspect of the embodiments, in any one of the eighth aspect to the tenth aspect, the OCT apparatus includes a movement mechanism (150) configured to relatively move the optical system relative to the object to be measured. The optical condition changing step is performed to change the optical condition of the optical system by controlling the movement mechanism.

According to such an aspect, by relatively moving the optical system relative to the object to be measured, the optical condition of the optical system is changed to perform OCT on the object to be measured. Thereby, the image of the object to be measured can be acquired with good image quality regardless of the cross-sectional shape of the object to be measured, and the measurement time (imaging time) can be shortened.

The thirteenth aspect of the embodiments, in the ninth aspect or the tenth aspect, further includes an image forming step of forming an image of the object to be measured based on the OCT data. The optical condition changing step is performed to control the optical path length difference changing member based a position of the object to be measured in the image.

According to such an aspect, the optical path length difference between the measurement light and the reference light is changed in synchronization with the period of the slow scan using the OCT data acquired during scanning. Thereby, the image of the object to be measured can be acquired with good image quality.

In the fourteenth aspect of the embodiments, in any one of the eighth aspect to the tenth aspect, the fast scan axis is a circular scan axis, and the slow scan axis is a spiral scan axis.

According to such an aspect, the region near the center (scan center position of slow scan) can be scanned at high density in a short period of time, and the farther away the scan position is from the center, the lower the density of the scan becomes in a longer period of time, which allows the scan of a wide region including the site of interest with a simple control.

The fifteenth aspect of the embodiments is a program of causing a computer to execute each step of the method of controlling the OCT apparatus of any one of the eighth aspect to the tenth aspect.

According to such an aspect, the optical condition(s) of the optical system can be changed in synchronization with the period of the slow scan using the OCT data acquired during scanning. Thereby, the image of the object to be measured can be acquired with good image quality without degrading the image quality due to misalignment of the scan position during scanning, etc. In particular, in case of imaging the object to be measured at a wide angle, the image of the object to be measured can be acquired with good image quality without performing pre-scan.

The configuration described above is only an example for suitably implementing the present invention. Therefore, any modification (omission, substitution, addition, etc.) within the scope of the gist of the present invention can be appropriately applied. The configuration to be employed is selected according to the purpose, for example. In addition, depending on the configuration to be applied, it is possible to obtain the actions and effects obvious to those skilled in the art and the actions and effects described in this specification.

### [Explanation of symbols]

1, 1a Ophthalmic apparatus
3 Display apparatus
41 Optical path length changing unit
45 OCT focusing lens
47 VCC lens
47A VCC driver
70 Wavefront aberration correction optical system
100 OCT unit
103, 118 Polarization controller
114 Corner cube
114A Reference driver
200 , 200a Arithmetic control unit
210, 210a Controller
211, 211a Main controller
212, 212a Storage unit
220 Image forming unit
230 Data processor
231 Image synthesizing unit
232 Analyzer
232A Image position identifying unit
232B Image quality evaluation value calculator
240A Display unit
E Eye to be examined
SAR1 Central region
SAR2 Intermediate region
SAR3 Peripheral region
LC Interference light
LR Reference light
LS Measurement light

## Claims

1. An optical coherence tomography apparatus, comprising:
an optical system configured to allow an optical condition to be changed, including an optical scanner, and configured to acquire optical coherence tomography data of an object to be measured by scanning the object to be measured with measurement light using the optical scanner; and
a controller configured to control the optical scanner so as to scan the object to be measured with the measurement light along a fast scan axis with reference to a reference position moved along a slow scan axis, wherein
the controller is configured to change the optical condition in synchronization with a period of the slow scan along the slow scan axis.

2. The optical coherence tomography apparatus of claim 1, wherein
the optical system includes:
an interference optical system configured to split light from a light source into the measurement light and reference light, to project the measurement light having traveled through a measurement optical path, on which one or more optical elements are arranged, onto the object to be measured, and to detect interference light between returning light from the object to be measured and the reference light having traveled through a reference optical path; and
an optical path length difference changing member configured to change a difference between an optical path length of the measurement light and an optical path length of the reference light, wherein
the controller is configured to change the optical condition of the optical system by controlling at least one of the one or more optical elements or the optical path length difference changing member.

3. The optical coherence tomography apparatus of claim 2, wherein
the one or more optical elements includes a focusing lens or an aberration correction device.

4. The optical coherence tomography apparatus of claim 2 or 3, further comprising:
an image forming unit configured to form an image of the object to be measured based on the optical coherence tomography data; and
an image quality evaluation value calculator configured to calculate an evaluation value of image quality of the image of the object to be measured, wherein
the controller is configured to control the one or more optical elements based on the evaluation value.

5. The optical coherence tomography apparatus of any one of claims 1 to 3, further comprising
a movement mechanism configured to relatively move the optical system relative to the object to be measured, wherein
the controller is configured to change the optical condition of the optical system by controlling the movement mechanism.

6. The optical coherence tomography apparatus of claim 2 or 3, further comprising
an image forming unit configured to form an image of the object to be measured based on the optical coherence tomography data, wherein
the controller is configured to control the optical path length difference changing member based a position of the object to be measured in the image.

7. The optical coherence tomography apparatus of any one of claims 1 to 3, wherein
the fast scan axis is a circular scan axis, and
the slow scan axis is a spiral scan axis.

8. A method of controlling an optical coherence tomography apparatus including an optical system configured to allow an optical condition to be changed, including an optical scanner, and configured to acquire optical coherence tomography data of an object to be measured by scanning the object to be measured with measurement light using the optical scanner, the method comprising:
a control step of controlling the optical scanner so as to scan the object to be measured with the measurement light along a fast scan axis with reference to a reference position moved along a slow scan axis; and
an optical condition changing step of changing the optical condition in synchronization with a period of the slow scan along the slow scan axis.

9. The method of controlling the optical coherence tomography apparatus of claim 8, wherein
the optical system includes:
an interference optical system configured to split light from a light source into the measurement light and reference light, to project the measurement light having traveled through a measurement optical path, on which one or more optical elements are arranged, onto the object to be measured, and to detect interference light between returning light from the object to be measured and the reference light having traveled through a reference optical path; and
an optical path length difference changing member configured to change a difference between an optical path length of the measurement light and an optical path length of the reference light, wherein
the optical condition changing step is performed to change the optical condition of the optical system by controlling at least one of the one or more optical elements or the optical path length difference changing member.

10. The method of controlling the optical coherence tomography apparatus of claim 9, wherein
the one or more optical elements includes a focusing lens or an aberration correction device.

11. The method of controlling the optical coherence tomography apparatus of claim 9 or 10, further comprising:
an image forming step of forming an image of the object to be measured based on the optical coherence tomography data; and
an image quality evaluation value calculation step of calculating an evaluation value of image quality of the image of the object to be measured, wherein
the optical condition changing step is performed to control the one or more optical elements based on the evaluation value.

12. The method of controlling the optical coherence tomography apparatus of any one of claims 8 to 10, wherein
the optical coherence tomography apparatus includes a movement mechanism configured to relatively move the optical system relative to the object to be measured, and
the optical condition changing step is performed to change the optical condition of the optical system by controlling the movement mechanism.

13. The method of controlling the optical coherence tomography apparatus of claim 9 or 10, further comprising
an image forming step of forming an image of the object to be measured based on the optical coherence tomography data, wherein
the optical condition changing step is performed to control the optical path length difference changing member based a position of the object to be measured in the image.

14. The method of controlling the optical coherence tomography apparatus of any one of claims 8 to 10, wherein
the fast scan axis is a circular scan axis, and
the slow scan axis is a spiral scan axis.

15. A program of causing a computer to execute each step of the method of controlling the optical coherence tomography apparatus of any one of claims 8 to 10.
